# EUROPEAN PATENT SPECIFICATION

(11) **EP 1 940 348 B1**
(45) Date of publication and mention of the grant of the patent: **14.09.2011**
(21) Application number: 06791478.8
(22) Date of filing: 20.10.2006
(51) Int. Cl.: A61K 8/66, A61K 38/44, A61Q 11/00, A23L 3/3571, C09D 5/14, C09D 5/16, A01N 63/00

(54) **A COMPOSITION COMPRISING A COUPLED ENZYME SYSTEM**
ZUSAMMENSETZUNG MIT EINEM GEKOPPELTEN ENZYMSYSTEM
COMPOSITION COMPRENANT UN SYSTEME D'ENZYME COUPLE

(30) Priority: 21.10.2005 DK 200501474
(43) Date of publication of application: 09.07.2008
(73) Proprietor: DANISCO A/S, 1001 Copenhagen K. (DK)
(72) Inventor: RAND, Thomas, DK-2605 Brøndby (DK); MADRID, Susan, Mampusti, DK-2970 Vedbæk (DK)
(74) Representative: Inspicos A/S
(86) International application number: PCT/DK2006/000590
(87) International publication number: WO 2007/045251

(56) References cited:
- EP-A- 1 157 684
- EP-A1- 0 451 972
- WO-A-97/06775

## Description

### FIELD OF THE INVENTION

The present invention relates to a composition comprising a coupled enzyme system for the rapid and efficient production of hydrogen peroxide by the coupling of a first enzyme system capable of hydrogen peroxide generation, to a second enzyme system which utilizes the non hydrogen peroxide product of the first enzyme system; and optionally is capable of generating further hydrogen peroxide.

### BACKGROUND OF THE INVENTION

Oral malodour and discoloration of the teeth are conditions that affect many people. Malodour of the oral cavity is also known as halitosis or bad breath. It is generally believed that the cause of this condition is due to the presence of anaerobic bacteria, especially gram-negative anaerobic bacteria, in the mouth. These bacteria will generate volatile sulphur compounds (VSC) which are known to cause breath malodour.

Dental plaque is a yellowish bio-film that builds up on the teeth. If not removed regularly, it can lead to dental cavities (caries), gingivitis and peridontitis and eventually tooth loss. The microorganisms that form the biofilm are almost entirely bacterial, with the composition varying by location in the mouth. Periodontal disease affects the periodontum, which is the investing and supporting tissues surrounding a tooth (*i.e.*, the periodontal ligament, the gingiva, and the alveolar bone). Gingivitis and periodontitis are inflammatory disorders of the gingiva and the deeper periodontal tissues, respectively.

Today consumers are very interested in making their teeth whiter. People with whiter teeth are considered as having more personal confidence and better social acceptance. Teeth comprise both an inner dentin layer and an outer hard enamel layer. The enamel layer protects the inner dentin layer and live tissue and serves as the contact surface for mastication of solid food. The enamel layer is generally translucent and slightly off-white in colour. It is also considered porous since the hydroxy apatite crystals that comprise the enamel form microscopic hexagonal rods or prisms having microscopic pores or channels between them. As a result of this porous structure, staining agents and discolouring substances, such as antibiotics, foods containing colouring materials, coffee, cola, tea, tobacco, etc., can permeate the enamel and change its surface to appear yellow or brownish in colour.

While good oral hygiene, as achieved by brushing the teeth with a cleansing dentifrice, may help reduce the incidence of stain, gingivitis, plaque, periodontal disease, and/or breath malodour, it does not necessarily prevent or eliminate their occurrence. Microorganisms contribute to both the Initiation and progression of gingivitis, plaque, periodontal disease, and/or breath malodour. Thus, in order to prevent or treat these conditions, these microorganisms must be suppressed by some means other than simple mechanical scrubbing. In addition, simple mechanical scrubbing will not be entirely effective to remove all stain types and/or whiten the teeth.

Enzymes which belong to EC class 1.1.3. are oxidoreductases which utilise oxygen as acceptor, and CH-OH groups are the donor. The capability of such oxygen oxidoreductases to generate hydrogen peroxide, which has an antimicrobial effect, has been utilized to improve the storage stability of certain food products including cheese, butter and fruit juice as it is disclosed in JP-B-73/016612. It has also been suggested that oxidoreductases may be potentially useful as oxygen scavengers or antioxidants in food products. Tooth bleaching composition comprising oxidoreductase(s) is described in US 6,379,653, where bleaching of teeth was obtained by treatment with glucose oxidase. Glucose oxidase is highly specific for glucose and requires presence of this cariogenic sugar that degrades in the mouth to compounds responsible for cavities.

WO97/06775 discloses oral compositions which comprise at least one oxidoreductase. The oxidoreductases considered by WO97/06775 include enzymes within the enzyme classes comprising oxidases including E.C. 1.1.3. E.C. 1.2.3, E.C. 1.3.3, E.C. 1.4.3, E.C. 1.5.3, E.C. 1.7.3, E.C. 1.8.3, E.C. 1.9.3, laccases and related enzymes comprised in E.C. 1.10.3 and peroxidases in E.C. 1.11. Substrates that are not cariogenic, such as amino acids, alcohol, sugar alcohol, such as xylitol and sorbitol are considered as suitable substrates for oxidoreductases. A specific xylitol oxidase considered is the xylitol oxidase disclosed in JP 80892242, which is reported to oxidize xylitol, D-sorbitol, D-galactitol, D-mannitol and D-arabinitol in the presence of oxygen.

The inclusion of certain oxidative enzymes in oral compositions such as toothpastes, mouthrinses and dentifrices can reduce plaque and gingivitis. The enzymes that have been used include as their active ingredients, amyloglucosidase and glucose oxidase. These produce hydrogen peroxide from dietary fermentable carbohydrates which in turn converts thiocyanate to hypothiocyanite in the presence of salivary lactoperoxidase. The resultant hypothiocyanite acts as a bacterial inhibitor by interfering with cell metabolism. Sorbitol oxidase is known e.g. from Hiraga K. et al. "Molecular cloning and expression of a gene encoding a novel sorbitol oxidase from Streptomyces sp. H-7775.";Biosci. Biotechnol. Biochem. 62:347-353(1998) describing cloning and expression of sorbitol oxidase from *Streotomyces sp.*

The majority of sugar substitutes approved for food use are artificially synthesized compounds. However, some natural sugar substitutes are known - including sorbitol and xylitol, which are found In berries, fruit, vegetables and mushrooms. Although natural, they may be produced synthetically in bulk food production, to lower production costs. Both xylitol and sorbitol are used in oral care compositions such as toothpaste or chewing gum to give a sweet taste and, in the case of xylitol, for decreasing lactic acid production and increasing saliva production (Hayes C. J Dent Educ. 65(10): 1106-1109 2001).

Even though a great deal of research has been carried out in order to find compositions useful for the treatment and/or prevention of gingivitis, plaque, periodontal disease, and/or breath malodor and/or for the whitening of teeth, additional efficacious compositions and methods of treatment for these purposes are still desirable.

Detergents for laundry and dish washing consist of complex mixtures of a wide variety of ingredients, which typically include a number of components such as ionic and non-ionic surfactants, solvents, builders, perfumes, enzymes, and bleaching components. In such complex mixtures, storage stability problems, particularly of enzymes, are well known. In some cases, stability problems are related to the physical stability of the detergent, while in other cases, it relates to the functional stability of the individual ingredients in the detergent. Bleaching agents such as percarbonates and perborates, are commonly used in powder detergents where they, together with bleach activators (e.g., tetra acetylethylenediamine (TAED) and nonanoyloxybenzenesulfonate (NOBS)), act to generate peracids (e.g. peracetic acid), hydrogen peroxide, and/or other related species upon addition of water during the wash cycle. The peracids or the other active oxygen species then act to bleach or lighten certain stains on the fabric or dishware. However, there is no ideal bleaching system available for use in aqueous liquid formulations. In addition, there is a need for the production of bleaching agents (e.g., active oxygen species, peroxide, and peracids) upon dilution of the detergent in the laundry wash liquor to bleach and/or lighten stains.

Enzymes such as oxidases are in particular susceptible to storage stability issues in liquid detergent formulation. This prevents their widespread use in fabric and house hold cleaning compositions that Involve bleaching action. Maintaining the oxidase enzymatic activity in detergents during storage has been a challenge, especially in detergents that also contain oxidase substrate components. The presence of both oxidase and oxidase substrate results in the in situ generation of peroxygen. This results in decreased enzyme stability due to oxidation of the enzymes both in liquid and dry formulations. Peroxides damage enzymes by various mechanisms such as oxidizing some of the amino acid residues in the enzyme or by interacting with the enzymes' cofactors. This often results in a gradual loss of activity. In dry detergent formulations enzymes can be stabilized by (e.g. encapsulation of the enzymes as described In WO 96/02623.

### SUMMARY OF THE INVENTION

The present invention relates to a composition comprising a first oxidase, a first substrate, and at least one further oxidoreductase, wherein, the first substrate is oxidisable by the first oxidase to form hydrogen peroxide and a second substrate; and the second substrate is convertible by the at least one further oxidoreductase to form a product; wherein the second substrate is oxidisable by the further oxidoreductase to form hydrogen peroxide and said product and the use thereof for whitening and/or bleaching of e.g. teeth, skin, hair, textiles or paper, an oral care product, and a non-therapeutic method for whitening and/or bleaching of teeth, the use as a preservative and as anti-Microbial agent, use in cosmetics, in detergents, in paints, In food and feed, In food and feed production and preparation, and in pesticides.

The present invention is based upon a surprising synergy which the present inventors have found when a hydrogen peroxide generation system comprising a first oxidase such as a polyol oxidase, and a first substrate, such as a polyol, is coupled to a further oxidoreductase enzyme system which utilizes the non hydrogen peroxide product generated by the first oxidase (*i.e.* the second substrate) optionally generating further hydrogen peroxide.

The coupling of the first oxidase and second oxidoreductase enzyme (systems) has been found to greatly enhance the efficiency of the hydrogen peroxide production from the first enzyme system, and can also result in production of hydrogen peroxide from both the first and second substrates. The effect is a considerably higher hydrogen peroxide generation and due to the surprising synergy between the first oxidase and the further oxidoreductase, a far higher rate of hydrogen peroxide compared to what would have been expected from the first oxidase enzyme system alone (or the further oxidoreductase enzyme system alone). It is as if the coupling of the further enzyme system 'turbo-charges' the first oxidase, forcing the very high level of hydrogen peroxide production.

The first enzyme is an oxidase, and is referred to as a first oxidase herein. A preferred oxidase is polyol oxidase, such as sorbitol oxidase.

The present invention provides detergent compositions comprising the composition, of the invention as well as methods for the use of the composition of the invention in liquid detergent compositions for bleaching and cleaning, for example of coloured food stains.

During the development of the present invention, it was surprisingly found that sorbitol oxidases were suitable for use in bleaching systems that avoided the disadvantages plaguing currently used bleaching systems.

The coupling of the first oxidase to a further oxidoreductase enzyme therefore allows full exploitation of the oxidative capacity locked up in the first substrate. The first oxidase appears to act as a 'key' which is surprisingly robust in a detergent environment, and, as disclosed herein, allows efficient and rapid production of hydrogen peroxide bleaching power, especially when coupled to a further oxidoreductase.

The present Invention provides in one aspect a composition comprising a first oxidase, a first substrate, and an oxidoreductase, wherein the first substrate is oxidisable by the first oxidase to form hydrogen peroxide and a second substrate, and the second substrate is oxidisable by the oxidoreductase to form hydrogen peroxide and a product.

In a further aspect the invention provides an oral care product comprising a composition according to the invention and ingredients used in oral care products.

In a further aspect the invention provides a cosmetic product comprising a composition according to the invention and one or more ingredients used in cosmetic products.

In a further aspect the invention provides a detergent product comprising a composition according to the invention and or more ingredients used in detergent products.

In a further aspect the invention provides a paint product comprising a composition according to the invention and or more ingredients used in paint products.

In a further aspect the invention provides a pesticide product comprising a composition according to the invention and or more ingredients used in pesticide products.

In a further embodiment, the invention provides for a bleaching or whitening product, such as a bleaching or whitening product for bleaching or whitening external mammalian tissue, such as skin, hair or teeth comprising a composition according to the invention and ingredients used In bleaching and whitening products suitable for application on external mammalian tissue.

In a further embodiment, the invention provides for a cosmetic method for bleaching or whitening of external mammalian tissue comprising contacting the external mammalian tissue with a composition according to the Invention or the product for bleaching and/or whitening external mammalian tissue according to the invention in an amount and duration suitable for bleaching and/or whitening the external mammalian tissue.

The Invention also provides for a medicament comprising a composition according to the invention.

The invention also provides for an edible beverage comprising a composition according to the invention, such as a fruit juice.

In one embodiment, the composition does not comprise the first substrate, but the first substrate is either naturally present or is added to the composition or application matrix.

The Invention further provides for a detergent or bleaching product comprising a composition according to the Invention and at least one further ingredient used In detergent or bleaching products.

The invention further provides for the non-therapeutic use of the composition according to the invention in an oral care product with beneficial teeth bleaching and/or whitening effects, and/or extended shelf life, and/or anti-microbial/anti-bacterial effects either prior to or during use.

The invention further provides for the non-therapeutic use of a composition according to the invention in an edible product with beneficial prebiotic effects when consumed by an individual mammal, and/or a prolonged shelf life.

The invention further provides for the non-therapeutic use of a composition according to the invention in a cosmetic product which has a prolonged shelf life, and/or is capable of bleaching and/or whitening external mammalian tissue, and/or has an anti-microbial/anti-bacterial effect when applied to the human skin.

The invention further provides for the use of a composition according to the Invention in a paint product which shows improved preservation either before or after application, and/or shows reduced anti-fouling.

The invention further provides for a method for the preparation of a composition comprising admixing a first oxidase and a first substrate, and at least one further oxidoreductase wherein the first substrate is oxidisable by the first oxidase such as the sorbitol oxidase, to form hydrogen peroxide and a second substrate, and the second substrate Is convertable by the at least one further oxidoreductase to form a product.

The composition may comprise a suitable matrix component or components to which the first oxidase and at least one further oxidoreductase are admixed. The first substrate may also be admixed Into the matrix component, or, in one embodiment form part or even the whole of the matrix component. The matrix component may therefore consist or comprise of the first substrate.

The invention further provides for a method for the preparation of a composition comprising admixing a first oxidase and a first substrate, and at least one further oxidoreductase, wherein the first substrate Is oxidisable by the first oxidase, such as the sorbitol oxidase, to form hydrogen peroxide and a second substrate, and the second substrate is oxidisable by the oxidoreductase to form hydrogen peroxide and a product.

The invention further provides for the use of a composition according to the invention, in the manufacture of a medicament for the treatment or prevention of a medical disorder selected from: gum disease, gingivitis, periodontal disease, irritable bowel syndrome, lactose Intolerance, colon cancer, high blood cholesterol, high blood pressure, hypertension, infection, inflammation and nutritional deficiencies.

The invention further provides for a method of generating hydrogen peroxide, the method comprising admixing a first oxidase and a first substrate, and at least one further oxidoreductase under conditions suitable for the generation of hydrogen peroxide from the oxidation of the first substrate due to the activity of the first oxidoreductase and optionally generation of further hydrogen peroxide from the oxidation of a second substrate due to the activity of the at least one further oxidoreductase wherein the second substrate is generated by the oxidation of the first substrate by the first oxidase, and wherein the second substrate is converted into a product by the at least one further oxidoreductase.

The invention further provides for a method of generating hydrogen peroxide, the method comprising admixing a first oxidase and a first substrate, and at least one further oxidoreductase under conditions suitable for the generation of hydrogen peroxide from both the oxidation of the first substrate due to the activity of the first oxidase, and the generation of hydrogen peroxide from the oxidation from a second substrate due to the activity of the at least one further oxidoreductase, wherein the second substrate is generated by the oxidation of the first substrate by the first oxidase.

In another aspect the invention provides the use of the composition according to the invention for whitening and/or bleaching.

In another aspect the invention provides a method for bleaching and/or whitening of teeth, comprising contacting the teeth with an oral care product comprising a composition according to the invention in an amount and time suitable for bleaching and/or whitening teeth.

The invention provides for the use of a composition according to the invention for whitening and/or bleaching teeth.

Although the main aspects of the invention refer to a coupled enzyme system, in some embodiments, such as the following embodiments, the invention provides for compositions which comprise a polyol oxidase and a first substrate, as referred to herein. The use of the polyol oxidase/first substrate enzyme system has been found to be highly beneficial in these applications, such as the generation of hydrogen peroxide from a non fermentable substrate, optionally without lowering the pH (e.g. like when not coupled to a further oxidoreductase system), and the anti-microbial/bacterial, anti-spoilage, bleaching and whitening characteristics thereby provided.

The invention provides for a paint composition comprising a polyol oxidase and a first substrate, wherein the first substrate is oxidisable by the polyol oxidase to form hydrogen peroxide.

The invention provides for a cosmetic composition comprising a polyol oxidase and a first substrate, wherein the first substrate is oxidisable by the polyol oxidase to form hydrogen peroxide.

The invention provides for a food or feed composition comprising a polyol oxidase and a first substrate, wherein the first substrate is oxidisable by the polyol oxidase to form hydrogen peroxide, such as a food or feed composition is selected from the group consisting of: Dairy products, such as milk, cream, cheese, whey; beverages, such as fruit juice,

The invention provides for a medicament composition comprising a polyol oxidase and a first substrate, wherein the first substrate is oxidisable by the polyol oxidase to form hydrogen peroxide, such as when the first substrate is sorbitol or preferably xylitol.

The invention provides for a pesticide composition comprising a polyol oxidase and a first substrate, wherein the first substrate is oxidisable by the polyol oxidase to form hydrogen peroxides.

### FIGURES:

Figure 1: The expression plasmid (pKB105-TAT-SOX-7775).
Figure 2: The plasmid "pKB105-CelA-Sox7775".
Figure 3: The Initial velocity of H₂O₂ production using the compositions with between 1x and 3000x excess of the further enzyme (oxidoreductase) compared to the first oxidase (SOX), as measured over 5 minutes in 300 uL ABTS assay. A dramatic synergy was seen with both the glucose oxidase and hexose oxidase further enzymes, with upto about 250 - 300% increase in hydrogen peroxide production rate seen.
Figure 4: The initial velocity of H₂O₂ production using the compositions with between 1× and 3000x excess of the further enzyme (oxidoreductase) compared to the polyol oxidase, as measured over 5 minutes in 300 µL ABTS assay. A dramatic synergy was seen with both the glucose oxidase and hexose oxidase further enzymes, especially at dosages greater than 1×, such as at least 2x dosage of the further enzymes compared to the polyol oxidase.
Figure 5a: pET 24a - sorbitol oxidase (H7775) expression vector.
Figure 5b. Expression of active Sorbitol oxidase in E.coli BL21(DE3) pLysS strain:
   a) Expression vector containing the 1.27 NdeI-BamHI fragment, encoding the *Streptomyces* H-7775 SOX synthetic gene.
   b) In gel overlay activity assay (PMS/NBT) using sorbitol as substrate. Negative control: Glucose oxidase (GOX) In lane 1, lanes 2-10 are cell lysates from the different transformants.

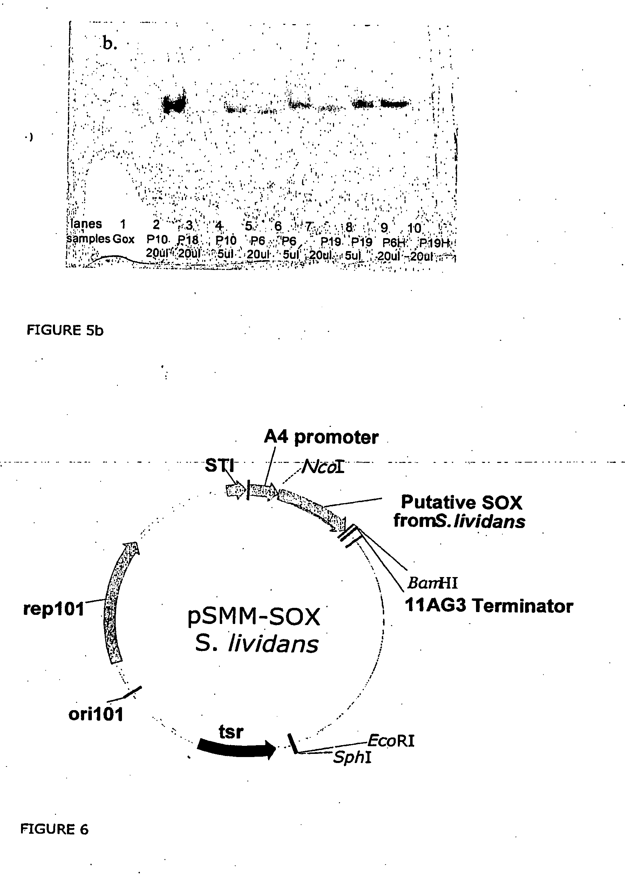

Figure 6. The construct for expression of the putative SOX gene in Streptomyces lividans strain g3s3. The putative SOX gene was cloned as Nco1-BamH1 PCR fragment and inserted.

### DETAILED DESCRIPTION OF THE INVENTION

The present invention relates in one aspect to a composition comprising a first oxidase such as a polyol oxidase (e.g. sorbitol oxidase), a first substrate, and a further oxidoreductase, wherein the first substrate is oxidisable by the first oxidase, to form hydrogen peroxide and a second substrate, and the second substrate is convertible by the further enzyme to produce a product.

The further enzyme is a further oxidoreductase, and the second substrate is oxidisable by the oxidoreductase to generate hydrogen peroxide and the (further) product.

The composition according to the invention Is applicable for all purposes where production of H₂O₂ Is needed *e.g.* in applications where bleaching and/or whitening is required or for antimicrobial purposes, and especially In products where non-toxic or environmentally acceptable ingredients are desired.

### The First Substrate

In the present context the term "first substrate" refers to a substrate which is oxidisable by first oxidase (such as sorbitol oxidase) to generate hydrogen peroxide and a second substrate.

In one aspect of the invention the first substrate is a polyol, such as one or more substrates selected from sugar alcohols such as selected from the group consisting of D-sorbitol, D-xylitol, D-mannitol, D-arabitol, glycerol, inositol, 1,3-propanediol, 1,3-butanediol, and 1,4-butanediol.

In one embodiment, the first substrate is one or more polyols selected from the group consisting of D-sorbitol or D-xylitol.

In one embodiment the first substrate is D-sorbitol.

It is recognized that sugars and sugar-alcohols that exist in D or L stereoisomers, it is the D form which is prevalent in nature, and are therefore preferred in terms of the first and second substrates as referred to herein.

In a further aspect of the invention the first substrate is a non-cariogenic sweetener such as selected from the group consisting of D-sorbitol or D-xylitol. In yet a further aspect the first substrate is D-sorbitol. D-sorbitol and D-xylitol are essentially non-cariogenic and are already used in oral care products e.g. In chewing gum as an artificial sweetener with beneficial results (Hayes C. J Dent Educ. 65(10): 1106-1109 2001).

In one aspect the first substrate is one or more sugar alcohol substrates selected from the group consisting of sorbitol, xylitol, maltitol, mannitol, galactitol, isomalt, lactitol, arabitol, and erythritol.

In one aspect the first substrate may be selected form the group consisiting of: ribitol, threitol, lyxitol, allitol, altritol, gulitol, iditol, talitol, pentitol and hexitol.

In one aspect the first substrate is one or more sugar alcohol substrates selected from the group consisting of sorbitol, xylitol, maltitol, mannitol, galactitol, isomalt, lactitol, arabitol, erythritol, glycerol, inositol, 1,2-propanediol, 1,3-butanediol, and 1,4-butanediol.

In a preferred aspect of the invention the first substrate is or comprises sorbitol.

In one aspect of the invention the first substrate is or comprises xylitol.

The polyol oxidase and further oxidoreductase are oxidases which are capable of generating peroxide (H₂O₂).

The level of polyol present in the composition accoridng to the invention will depend upon the application and the formulation used. For use in oral care products a high level of polyol may be used, where the polyol may be the major matrix ingredient in the composition. Polyols can also form a major component of cosmetic formulations. In such applications polyols may be added as humectants. Polyols may also be added to detergents such as soaps, where they can also have a humectant function or as a clarifying agent.

However, in some applications, such as in some paint and detergent applications the polyol may be added as a minor component, sufficient to provide enough first substrate for the generation of hydrogen peroxide, but not forming a major matrix component.

Therefore, for example, the level of first substrate present in the composition of the invention, prior to the oxidation into the second substrate may be between about 0.05% to about 80% w/w, such as between 0.1% and about 70% w/w.

Suitably, in one embodiment, the level of polyol present in oral care products may therefore be between about 1 to about 80% w/w, such as between about 10 to about 75% w/w, or such as between about 20 to about 70% w/w.

Suitably, in one embodiment, the level of polyol present in paint products may range from between about 0.01 to about 20 % w/w, such as from about 0.1 to about 10% w/w, such as from about 1 to about 5% w/w.

Suitably, In one embodiment, the level of polyol present in a cosmetic composition or products according to the invention may range from between about 1 to about 50% w/w, such as between about 5 to about 40% w/w, or such as between about 10 to about 40% w/w. US patent 7094395 discloses cosmetics which comprise about 8-32% polyol (humectants), such a range may also be used in the compositions of the present invention.

Suitably, In further embodiments, the level of polyol present in detergent products may range from between about 0.01% to about 40% w/w, such as between about 0.1% to about 30%, such as between about 1% to about 20%, such as between about 1% to about 10% or between about 1% and about 5%.

### The First Enzyme

The first enzyme is an oxidase enzyme, and is referred to as 'first oxidase' herein.

The first oxidase may be derived or Isolated from an organism selected form the group consisting of: *Streptomyces*, *Xanthomonas*, *Brevibacterium*, *Frankia*, *Nocardia*, *Janibacter*, *Burkholderia*, *Paracoccus*, *Chromabacterium*, *Thermobifida*, *Psuedomonas*, *Corynebacterium* and *Bacillus* species and their homologs

Suitable first oxidases may include enzymes which are categorized under an Enzyme Classification number (E.C.) selected from the group consisting of: EC 1.1.3.14 catechol oxidase , EC 1.1.3.18 secondary-alcohol oxidase, EC 1.1.3.41 xylitol oxidase, EC 1.1.3.13 alcohol oxidase, EC 1.1.3.19 4-hydroxymandelate oxidase, EC 1.1.3.20 longchain alcohol oxidase, EC 1.1.3.40 D-mannitol oxidase. EC 1.1.3.7 arylalcohol oxidase, EC 1.1.3.30 polyvinylalcohol oxidase, EC 1.1.3.21 glycerolalcoholoxidase, and EC 1.1.3.38 vannilylalcohol oxidase.

JP 80892242 discloses a xylitol oxidase which oxidises xylitol, D-sorbitol, D-galactitol, D-mannitol and D-arabinitol in the presence of oxygen.

A xylitol oxidase can be obtained from strains of *Streptomyces sp.* (e.g. *Streptomyces* IKD472, FERM P14339) having a pH optimum at 7.5, is stable at pH 5.5 to 10.5 and at temperatures up to 65°C; properties very well suited for the applications disclosed herein, such as oral care and detergent compositions and products.

In one specific embodiment, the first oxidase is not the xylitol oxidase which can be obtained from strains of *Streptomyces sp.* (e.g. *Streptomyces* IKD472, FERM P14339) having a pH optimum at 7.5, and which is stable at pH 5.5 to 10.5 and at temperatures up to 65°C.

During the development of the present invention, it was surprisingly found that polyol oxidases such as sorbitol oxidases were suitable for use in bleaching systems that avoided the disadvantages plaguing currently used bleaching systems. These sorbitol oxidases include enzymes isolated from such organisms as *Streptomyces* or *Xanthomonas* species and their homologs. However, it is not intended that the present invention be limited to these specific nor any particular sorbitol oxidase(s).

Sorbitol oxidase ("SOX" or "SoX") is an enzyme that catalyzes conversion of sorbitol to glucose and hydrogen peroxide. Sorbitol oxidases are known and used in various applications (*See e.g.*, Oda and Hiraga, Ann. NY Acad. Sci., 864:454-457 [1998]; and Yamashita et al J. Biosci. Bioengin., 89:350-360 [2000]). Sorbitol (D-glucitol, C₆H₁₄O₆, MW 182.2, CAS 50-70-4) is a commonly used in enzyme product formulations. Thus, sorbitol oxidase provides an attractive biobleaching agent for use in detergents that incorporate these sorbitol-containing enzyme product formulations.

In one embodiment, the first oxidase has a higher specific activity on sorbitol as compared to xylitol, such as at least about 1.5x, or at least about 2x, higher specific activity on sorbitol as compared to xylitol

In one embodiment, the first oxidase has a specific activity on sorbitol of at least about 5 units/mg.

The specific activity of the first oxidase on sorbitol and xylitol substrates may be determined *in vitro*, such as using the assays provided in the examples, or alternatively the specific activity may be determined *in situ*, within said oral care composition.

A preferred SOX is an oxidoreductase that uses covalently bound FAD as a cofactor for oxidation of sorbitol to glucose. This enzyme offers a unique opportunity for its potential use as a biobleach agent on its own, as well as used in combination with carbohydrate oxidases such as glucose oxidase and/or hexose oxidase (see WO 96/39851), (gluco)oligosaccharide oxidase and *M. nivale* carbohydrate oxidase (see WO99/31990).

An advantage of the use of such combinations is due to the fact that SOX converts sorbitol to glucose, which can then be converted to gluconate by glucose oxidase and/or hexose oxidase, thus generating two moles of hydrogen peroxide per mole of sorbitol, as illustrated below.

D-Sorbitol + O₂ → D-Glucose + H₂O₂

D-Glucose + O₂ → D-Gluconate + H₂O₂

A preferred glycerol oxidase (GLOX) is an enzyme found in the genera *Penicillium* and *Botrytis* (*See e.g.*, Lin et al Enz. Micro. Technol.,18:383-387 [1996]; and Uwajima et al, Agric. Biol. Chem.., 44:399-406 [1989]). This enzyme catalyzes the conversion of glycerol and oxygen to glyceraldehyde and hydrogen peroxide as shown below.

CH₂OH-CHOH-CH₂OH +O₂ → CH₂OH-CHOH-CHO +H₂O₂

Glycerol (glycerin, C₃H₈O₃, MW 92.09, CAS 56-81-5) is commonly used in enzyme product formulations, soap and detergent formulations, food and beverages, pharmaceuticals and is widely used in cosmetics and personal care applications. Thus, glycerol oxidase provides an attractive biobleaching agent for use in detergents that incorporate these glycerol-containing enzyme product formulations.

During the development of the present invention, sorbitol oxidase was isolated from *Streptomyces lividans*(SCO6147) (SEQ ID NO 2) and *Streptomyces* sp. H7775 (SEQ ID NO 1) (*See*, Hiraga et al., Biosci. Biotech. Blochem., 61:1699-1704 [1997]). The sorbitol oxidase was expressed both intracellularly and extracellularly from these organisms. The prosthetic group is a covalently bound FAD (1 mol of FAD to 1 mol of SOX). Thus, it is a flavoprotein, with typical absorption maxima at 276, 358, and 455 nm for the H7775 SOX, 345nm for the SCO6147 SOX (as expressed in *S*. *lividans*), which is indicative of a histidine-flavin linkage. Flavin is functionally involved in oxidation of sorbitol as observed by desired changes in UV-VIS spectra. FAD is very tightly bound with the protein and thus offers a stable enzyme for laundry applications.

The SOX gene was cloned and sequenced from *Streptomyces* species H-7775 (Genbank accession number AB000519).

The sorbitol oxidase gene from *Streptomyces* species H-7775 (Genbank accession number AB000519) comprises a 1260 bp open reading frame (ORF) encoding a protein having 420 amino acids with theoretical MW of 45,158 Daltons. The enzyme is stable for 24 hours at 30°C, between pH 7.5-10 with an optimum temperature of 50°C at pH 7.5. It is also heat stable up to 55°C. The nearest homolog identified for this enzyme is xylitol oxidase (51% homology). SOX is an efficient enzyme for multiple applications, including detergents, fabric care, home care, oral care (*e.g.*, dental whitening and/or cleaning), personal care, textile processing, food processing and industrial cleaning. In addition, numerous SOXs can catalyze other substrates such as xylitol, mannitol, arabitol, ribitol, erythritol, inositol, glycerol, propane diol, and butane diol. Thus, this enzyme uses a wider spectrum of substrates, providing flexibility in substrate usage in various applications.

Many of these first substrates, as provided herein, are present in typical detergent, oral care and cosmetic formulations or can be added to them.

The amino acid sequence of sorbitol oxidase from *Streptomyces sp.* H7775 Is known In the art and set forth in SEQ ID NO: 1.

As indicated above, the polyol oxidase such as the sorbitol oxidases utilised herein were found to be thermally stable and stable over a wide pH range. Indeed, the pH profile of the sorbitol oxidase used were found to be compatible with the pHs necessarily used in industry, as well as detergents and other cleaning agents.

In addition, the polyol oxidase such as the sorbitol oxidase provided by the present invention may preferably produce sugar such as glucose, *i.e.* an aldehyde product that can be further oxidized to gluconic acid, a carboxylic acid product, using other oxidases such as hexose oxidase or glucose oxidase releasing another molecule of hydrogen peroxide from starting substrate sorbitol. Similarly oxidation of polyols such as xylitol, arabitol, mannitol, by sorbitol oxidase, xylitol oxidase, mannitol oxidase with the assistance of atmospheric oxygen with formation of the corresponding sugar, such as xylose, arabinose, mannose, respectively as secondary substrate for further oxidation by other relevant oxidases such as hexose oxidase, xylose oxidase, pyranose oxidase, arabinose oxidase, and mannose oxidase is feasible.

For use in edible and oral care compositions, it is also advantageous to use enzymes being substantially active at pHs prevailing in the mouth, *i*.*e*. between pH 5.0 to 9.0, preferably between pH 6.0 to 8.5, especially between pH 6.4 to 7.5.

In one embodiment, the polyol oxidase is a pentitol or hexitol oxidase.

In one embodiment the polyol oxidase is not a triose oxidase, or is not a glycerol oxidase.

It is recognised that enzymes may have activity on more than one substrate. Therefore, when we refer to an enzyme by the name of the compound which it oxidises, for example sorbitol oxidase (sorbitol) or hexose oxidase (hexose) or glucose oxidase (glucose), the name refers to either the classification which the enzyme has been given, such as the EC number, or the name the enzyme is referred to in the art, or the predominant activity as compared to the substrate for which the enzyme has the highest specific activity. In this respect, a sorbitol oxidase has a higher specific activity on sorbitol than, for instance xylitol. Suitably, sorbitol oxidase may, in one embodiment, also catalyse the oxidation of several polyols including at least two of the group consisting of D-sorbitol, D-xylitol, D-mannitol, D-arabitol, glycerol, inositol, 1,2-propanediol, 1,3-butanediol, and 1,4-butanediol.

In one embodiment the polyol oxidase is selected from the group consisting of ribitol oxidase, threitol oxidase, xylitol oxidase, allitol oxidase, altritol oxidase, gulitol oxidase, iditol oxidase, talitol oxidase, pentitol oxidase and hexitol oxidase.

In one embodiment the polyol oxidase has a ratio of specific activity on the polyol it is named after, compared to the specific activity on an alternative substrate, as listed herein as either said frist substrate or said second substrate, excluding the substrate the polyol is named after, of greater than 1, such as greater than about 1.5, such as greater than about 2, such as greater than about 3, such as greater than about 4, such as greater than about 5, such as greater than about 10. In one embodiment the polyol oxidase has a ratio of specific activity on sorbitol compared to the specific activity on an alternative substrate, of greater than 1, such as greater than about 1.5, such as greater than about 2, such as greater than about 3, such as greater than about 4, such as greater than about 5, such as greater than about 10, wherein the alternative substrate is selected from the group consisting of: a sugar such as maltose, hexose, glucose, mannose, galactose, isomaltulose, lactose, arabinose, erythrose, pentose, xylose and triose, preferably glucose; a triose polyol, such as glycerol; and xylitol.

In one embodiment the polyol oxidase is not xylitol oxidase.

In one embodiment, the first oxidase, such as the polyol oxidase exhibits a higher activity on sorbitol than xylitol, such as at least one and a half times as much activity, such as at least two times as much activity. In the same embodiment or in a different embodiment, the polyol oxidase has no more than three times the activity on sorbitol as compared to xylitol.

In one embodiment, the first oxidase, such as the polyol oxidase is selected from the group consisting of: sorbitol oxidase, xylitol oxidase, maltitol oxidase, mannitol oxidase, galactitol oxidase, isomalt oxidase, lactitol oxidase, arabitol oxidase, arabitol oxidase and erythritol oxidase.

In one embodiment, the first oxidase, such as the polyol oxidase has a (specific) oxidase activity of at least about 5 units/g protein when using the respective (e.g. polyol) substrate, such as a substrate selected form the group consisting of: Sorbitol, xylitol, maltitol, mannitol, galactitol, isomalt, lactitol, arabitol, arabitol and erythritol.

A preferred first oxidase, such as the polyol oxidase is sorbitol oxidase, or an enzyme which exhibits sorbitol activity.

In one embodiment the first oxidase, such as the polyol oxidase is xylitol oxidase, or exhibits xylitol oxidase activity.

In one embodiment the first oxidase, such as the polyol oxidase is a glycerol oxidase, or comprises glycerol oxidase activity.

In one embodiment, the first oxidase, such as the polyol oxidase has a ratio of specific activity on the respective polyol to the corresponding sugar of greater than 1, such as at least about 1.5, such as at least about 2, such as at least about 3, such as at least about 4, such as at least about 5, such as at least about 10. In one embodiment, the first oxidase, such as the polyol oxidase has a ratio of specific activity on the respective polyol to glucose of greater than about 1, such as at least about 1.5, such as at least about 2, such as at least about 3, such as at least about 4, such as at least about 5, such as at least about 10.

In one embodiment, the first oxidase, such as the polyol oxidase, such as the sorbitol oxidase or xylitol oxidase is derived or obtained from a strain of *Streptomyces, such as Streptomyces, coelicolor, or Streptomyces sp.* IKD472.

In one embodiment, the first oxidase, such as the polyol oxidase, such as the sorbitol oxidase is derived from or obtained from a strain of *Streptomyces coelicolor.*

In a preferred embodiment, first oxidase, such as the the polyol oxidase, such as the sorbitol oxidase, is a polypeptide consisiting of, or derived from SEQ ID NO 1 or SEQ ID NO 2 and homologues, variants or fragments thereof.

In one embodiment, using the assay provided by Example 6, the polyol oxidase has activity on D-sorbitol, D-xylitol, D-mannitol, D-ribitol, myo-inositol, and glycerol. In a further embodiment, which may be the same or different, the polyol oxidase has activity on 1,3 propanediol and/or 1,2 propanediol.

In one embodiment, using the assay provided by Example 6, the polyol oxidase does not have activity on propylene glycol and/or ethylene glycol.

Sorbitol oxidase (SOX) may be obtained from suitable microorganisms such as *Streptomyces.* Hiragi K. et al. (Biosci. Biotechnol. Biochem. 62:347-353(1998)) describes production of recombinant sorbitol oxidase in *E.coli.* Other sources are described In *e.g.* US patent 5,741,687 and US 5,472, 862 wherein a microorganism from the soil of Sekigahara-cho, Fuwa-gun, Gifu Prefecture, Japan, from the genus *Xanthomonas,* such as *Xanthomonas maltophilla* TE3539 (FERM BP-4512) is described. Other examples of polyol oxidases are mannitol oxidase (EC 1.1.3.40) from the snails *Helix aspersa* and *Arlon ater* that catalyzes the oxidation of D-arabinitol, D-mannitol and, to a lesser extent, D-glucitol (sorbitol), and xylitol oxidase ( EC 1.1.3.41) from *Streptomyces coelicolor* that oxidises D-xylitol to xylose and H₂O₂ and D-sorbitol to glucose and H₂O₂.

The first oxidase, such as the polyol oxidase, such as a sorbitol or xylitol oxidase may be obtained from a microbial source such as a bacterium or a fungus. In particular from bacteria classified into the class Actinobacteria and order Actinomycetales.

The first oxidase, such as the polyol oxidase, such as a sorbitol or xylitol oxidase may be obtained from different species of *Streptomyces, Xanthomonas, Brevibacterium, Frankia, Nocardia, Janibacter, Burkholderia, Para coccus, Chromabacterium, Thermobifida, Psuedomonas, Corynebacterium* and *Bacillus* species and their homologs.

The first oxidase, such as the polyol oxidase, such as a sorbitol or xylitol oxidase may be obtained from a strain of *Streptomyces,* preferably from a strain of *Streptomyces coelicolor* or *Streptomyces* sp. IKD472 (Yamashita, Mitsuo et al., Journal of Bioscience and Bioengineering (2000), 89(4), 350-360), and *Streptomyces sp.* H-7775. (Hiraga, Kazumi et al., Bioscience, Biotechnology, and Biochemistry (1997), 61(10), 1699-1704.).

JP 09206072 discloses a sorbitol oxidase of *Streptomyces* and its production method and use, which may also be used in the present invention.

JP 06169764 discloses a sorbitol oxidase of *Xanthomonas* which may also be used in the present invention.

In one aspect of the invention the first oxidase, such as the polyol oxidase, such as the sorbitol oxidase is derived from a strain of *Streptomyces.*

In a further aspect the first oxidase, such as the polyol oxidase e.g. the sorbitol oxidase is produced by recombinant methods.

In one embodiment, first oxidase and the further oxidoreductase are active at ambient temperature.

In one embodiment, first oxidase and the further oxidoreductase are active at mouth or body temperature, such as about 37°C.

Although it is recognised that the skilled person will dose the first oxidase at a level suitable (an effective amount) for the required purpose, it is envisaged that In one embodiment the first oxidase is at a level of between about 0.1 and about 200,000 units per kg, such as between about 0.1 and about 100,000 units per kg, such as between about 1 and about 100,000 units per kg, such as between about 5 and about 50,000 units per kg, such as between about 10 and about 20,000 units per kg, such as between about 100 and about 10,000 units per kg. Other suitable ranges, for use In some embodiment, include between about 10 and about 1,000 units per kg, or between about 1 and about 10,000 units per kg.

In one embodiment, refering to an oral care composition between 1 and 10000U (units)/100 g first oxidase may be used. Dosages around 5-20 units per 100g are also considered appropriate for some embodiments.

For use in food and feed compositions, the above dosage ranges may also be appropriate.

In one embodiment, refering to a paint composition between about 10 and about 10000U/100 g first oxidase such as the polyol oxidase may be used. Dosages around 10-50 units per 100g are also considered appropriate for some embodiments.

In one embodiment, refering to a cosmetic composition between about 1 and about 10000U/100 g first oxidase such as the polyol oxidase may be used. Dosages around 5-20 units per 100g are also considered appropriate for some embodiments.

In one embodiment, refering to detergent composition between about 0.1 and about 10000U/100 g first oxidase such as the polyol oxidase may be used. Dosages around 5-100 units per 100g are also considered appropriate for some embodiments.

### The Second Substrate

The second substrate is cabable of being converted by the (at least one) further oxidoreductase to the product. The second substrate is, preferably oxidisable by the further oxidoreductase to generate further hydrogen peroxide and the product.

In the present context the term "second substrate" refers to a substrate which is a result of the oxidation of the first substrate and is convertable by the further oxidoreductase to form the product,

In a preferable embodiment, the term "second substrate" refer to a substrate which is a result of the oxidation of the first substrate by the first oxidase and is oxidisable by the further oxidoreductase to form hydrogen peroxide and the product.

The second substrate is, in one aspect, one or more sugars, such as sugars selected from the group consisting of glucose, xylose, maltose, mannose, galactose, isolmaltulose, lactose, arabinose and erythrose.

The second substrate is, in one aspect, one or more sugars, such as sugars selected from the group consisting of ribose, lyxose, allose, altrose, gulose, idose, and talose.

The second substrate may therefore be, or comprise glucose, for example when the first substrate is or comprises sorbitol.

Due to the remarkable synergy between the first oxidase, such as the polyol oxidase and the further oxidoreductase, the steady state level of the second substrate is typically low. In oral care products, as well as preservative applications, for example in paint or cosmetics, the low level of the second substrate may be highly advantageous, particularly when the second substrate is a fermentable or cariogenic sugar, *i.e*. compounds which can encourage or 'feed' the growth of detrimental organisms such as micro-organisms/bacteria (e.g. cariogenic bacterial In the oral cavity), or algae and barnicales (fouling on maritime paint for example). Therefore, not only does the invention provide a highly efficient and voluminous production of hydrogen peroxide, it can also provide a system where there is a minimal level of fermentable substrates, reducing the likelyhood of undesirable growth of detremental organisms.

In one embodiment, the level of the second substrate, as generated by the first oxidase, such as one or more fermentable or carlogenic sugars, present in the composition according to the invention is less than about 50%, such as less than about 25%, such as less than about 10%, such as less than about 5%, such as less than about 1%, such as less than about 0.5%, such as less than about 0.1%, such as less than about 0.05%, such as less than about 0.01% of the (initial) level of polyol present in the composition, as measured by a molar ratio.

In a preferred embodiment the second substrate Is a sugar.

In one embodiment the sugar is a monosaccharide hexose, such as a hexose selected from the group consisting of glucose, fructose, galactose, mannose, sorbose.

In one embodiment the sugar Is a monosaccharide pentose, such as a pentose selected from the group consisting of ribose, arabinose, xylose or lyxose.

In one embodiment the sugar is a triose, such as either aldotriose or ketotriose.

In one embodiment the sugar is a disaccharide such as sucrose or maltose,

### The Further Enzyme

The (at least one) further oxidoreductase is characterised in that it is capable of converting the second substrate to produce a product.

The further oxidoreductase is not the same enzyme, or enzymatic entity, as the first oxidase. However, In one embodiment, the further oxidoreductase may be attached to the first oxidase for example the first and further enzymes may be co-expressed as a polyprotein.

The further oxidoreductase may be any enzyme which, within the composition according to the invention, or in the applications disclosed herein, is capable of converting the second substrate into a product, the presence of which does not detrimentally affect the generation of hydrogen peroxide from the oxidation of the first substrate by the first oxidase.

Examples of suitable further oxidoreductase include glucose dehydrogenase (E.C: 1.1.1.118) which, when supplied with NAD+, results in the production of D-glucono-1,5-lactone + NADH; glucose 1-dehydrogenase (E.C: 1.1.1.119) (NADP(+)), which, when supplied with NADP+, results in the production of D-glucono-1,5-lactone + NADPH; glucokinase (E.C. 2.7.1.2, which phosphorylates the COOH group of glucose to produce glucose-6-phosphate; glucokinase, which converts D-glucose + D-fructose <=> D-gluconolactone + D-glucitol.

Therefore in one embodiment, the composition may comprise further compounds which are utilised by the further oxidoreductase with the second substrate to produce the product(s) - suitably such further compound may, for example, be selected form the group consisting of NAD+, NADP+, or fructose.

The further enzyme is a (at least one) further oxidoreductase (also refered to as oxidoreductase).

The further oxidoreductase is preferably an oxidase other than a polyol oxidase. The further oxidoreductase is not the polyol oxidase referred to herein within the context of the first oxidase.

Oxidoreductases are enzymes belonging to EC class 1.x.x.x. In the present invention the oxidoreductases utilise an oxygen acceptor, such as CH-OH or an aldehyde or oxo (EC 1.2.3.x).

In a preferred embodiment, oxidoreductases in the present context are enzymes belonging to EC class 1.1.3. acting with oxygen as acceptor. For example the hexose oxidase (D-hexose:O₂-oxidoreductase, EC 1.1.3.5) is an enzyme which in the presence of oxygen is capable of oxidizing D-glucose and several other reducing sugars including maltose, lactose and cellobiose to their corresponding lactones with subsequent hydrolysis to the respective aldoblonic acids upon formation of hydrogen peroxide. The oxidation catalyzed by hexose oxidase on glucose and galactose can e.g. be illustrated as follows:

D-Glucose + O₂ → δ-D-gluconolactone + H₂O₂, or

D-Galactose + O₂ → γ-D-galactogalactone + H₂O₂

In another aspect of the invention the oxidoreductase is one or more selected from the group consisting of hexose oxidase, glucose-oxidase, carbohydrate oxidase, and oligosaccharide oxidase such as (gluco)oligosaccharide oxidase. In a further aspect of the invention the oxidoreductase is one or more enzymes that catalyse oxidation of sugars such as selected from the group consisting of a carbohydrate oxidase, (gluco)oligosaccharlde oxidase, pyranose oxidase a hexose oxidase or a glucose oxidase. In a further aspect of the invention the oxidoreductase is a glucose oxidase and/or a hexose oxidase. In yet a further aspect of the invention the oxidoreductase is a hexose oxidase.

In a preferred embodiment, the oxidoreductase is a sugar oxidase, such as a sugar-oxidase selected form the group consisting of: carbohydrate oxidase, oligosaccharide oxidase, maltose oxidase, hexose oxidase, glucose oxidase, mannose oxidase, galactose oxidase, isolmaltulose oxidase, lactose oxidase, arabinose oxidase, erythrose oxidase, pentose oxidase, xylose oxidase, triose oxidase,

In one embodiment, the sugar-oxidase is selected form the group consisting of: EC 1.1.3.4 glucose oxidase, EC 1.1.3.5 hexose oxidase, EC 1.1.3.9 galactose oxidase, EC 1.1.3.10 pyranose oxidase, EC 1.1.3.11 L-sorbose oxidase, and EC 1.1.3.40 D-mannitol oxidase. Suitable oxidases may be identified under the Enzyme Classification number E.C. 1 (Oxidoreductases) in accordance with the recommendations (1992) of the International Union of Biochemistry and Molecular Biology (IUBMB)) which are enzymes catalysing oxidoreductions, in particular oxidases listed under E.C. 1.1.3. or E.C. 1.2.3, *i.e*. oxidases acts on molecular oxygen (0₂) and yield peroxide (H₂O₂), utilising either CH-OH or an aldehyde or oxo group as a donor.

A suitable glucose oxidase may originate from *Aspergillus sp.,* such as a strain of *Aspergillus niger,* or from a strain of *Cladosporiurn* sp. in particular *Cladosporium oxysporum,* especially *Cl.oxysporum* CBS 163 described in WO 95/29996 (from Novo Nordisk A/S).

Hexose oxidases from the red sea-weed *Chondrus crispus* (commonly known as Irish moss) (Sullivan and Ikawa, (1973),Biochim. Biophys. Acts, 309, p. 11-22; Ikawa, (1982), Meth. In Enzymol. 89, carbohydrate metabolism part D, 145-149) oxidises a broad spectrum of carbohydrates, such as D-glucose, D-galactose, maltose, cellobiose, lactose, D-glucose 6-phosphate, D-mannose, 2-deoxy-D-glucole, 2-deoxy-D-galactose, D-fucase, D-glucurnic acid, and D-xylose.

Also the red sea-weed *Iridophycus flaccidum* produces easily extractable hexose oxidases, which oxidise several different mono- and disaccharides (Bean and Hassid, (1956), J. Biol. Chem, 218, p. 425; Rand et al. (1972, J. of Food Science 37, p. 698-710).

The broad substrate spectrum of hexose oxidase is advantageous in the connection with tooth bleaching as the total amount of usable substrate (*i.e*. carbohydrate) present in the mouth is significantly greater than for related enzymes having more specific catalytic properties.

Carbohydrate oxidase form *Microdochium nivale* described in EP 1041890 acts on several sugars, including glucose, lactose and xylose as well as on oligosaccharides.

Another oxidoreductase capable of acting on several sugars and oligosaccharides is obtained from *Acremonium strictum* ( Lin, et al. *Biochemica and Biophysica Acta* 118(1991)). Its use in bakery applications is described in JP 11056219.

Other examples of suitable oxidoreductases are glucose oxidase (EC 1.1.3.4) and galactose oxidase (EC 1.1.3.9).

In one embodiment the oxidoreductase is one or more selected from the group consisting of a carbohydrate oxidase, a hexose oxidase or a glucose oxidase.

In one embodiment the oxidoreductase is a hexose oxidase.

Another example of a suitable oxidoreductase is hexose oxidase (HOX) (EC 1.1.3.5) which may be obtained by isolating the enzyme from several red algal species such as *Iridophycus flaccidum* (Bean and Hassid, 1956) and *Chondrus crispus* (Sullivan et al. 1973). In a preferred aspect of the invention HOX is obtained or prepared as described In WO 01/38544. HOX Is available from Danisco A/S as DairyHOX™

The dosage of the further oxidoreductase may be within the same ranges as referred to for the polyol oxidase above, although It is recognised that In one embodiment an excess of the further oxidase is added, as referred herein.

### The Enzyme Composition

It is preferred that purified enzymes, such as the sorbitol oxidase and/or further oxidoreductase are used, *i.e*. the enzymes are purified prior to being added to the composition of the invention. Enzyme purity is preferably determined using SDS-PAGE and densitometry. A purified enzyme is at least about 20% pure, such as at least about 30% pure, such as at least about 40% pure, such as at least about 50% pure. It is recognized that a purified enzyme may however be formulated with other proteins, for example mixed with protein stabilizers such-as BSA or other enzymes, the assessment of enzyme purifity therefore excludes proteins added to the enzyme after purification.

The rate of hydrogen peroxide production can be controlled by, for example, preparing a composition comprising specified ratio of the effective amount of the first oxidase compared to the further oxidoreductase. Typically, and as shown in the examples, an increasing excess of the further oxidoreductase results in increased rate of hydrogen peroxide production.

In one embodiment the molar quantity of total hydrogen peroxide produced (or producable), is greater than the molar quantity of the first substrate converted (or convertible) to the second substrate or the amount of the first substrate present. Suitably, the molar quantity of hydrogen peroxide produced (or producable), is, in one embodiment, between greater than 100% and less than or equal to 200% the molar quantity of the first substrate converted (or convertible) to the second substrate, or the amount of the first substrate present. The use of the present invention may therefore allow up to two molecules of hydrogen peroxide to be produced from a sorbitol first substrate, as compared to a single molecule of hydrogen peroxide from the use of a sorbitol oxidase enzyme alone. In one embodiment, the molar quantity of hydrogen peroxide produced (or producable) is up to two times the molar quantity of hydrogen peroxide which can be produced from a polyol oxidase system without a further oxidoreductase.

In one preferred embodiment, the amount of the at the least one further oxidoreductase compared to the amount of the oxidase, such as the polyol oxidase (e.g. sorbitol oxidase) present In the composition according to the invention Is greater than 1, as measured by the respective number of enzyme units present In said composition, such as greater than about 1.5, such as greater than about 2, such as greater than about 3, such as greater than about 5, such as greater than about 10, such as greater than about 20, such as greater than about 50, such as greater than about 100, such as greater than about 150, such as greater than about 200, such as greater than about 300, such as greater than about 500, such as greater than about 1000.

A further advantage of the composition according to the invention is the possibility of choosing a combination of enzymes and substrates generating the whitening agent hydrogen peroxide without accumulating cariogenic sweeteners or sugars as a product.

As an example SOX catalyses the oxidation of D-sorbitol to yield 1 mole of D-glucose and 1 mole of H₂O₂, The oxidoreductase, such as HOX, catalyses the oxidation of D-glucose to yield 1 mole of D-gluconic acid and 1 mole of H₂O₂. Combined use of SOX and HOX thus generates 2 moles of H₂O₂ from 1 mole of sorbitol oxidase substrate as illustrated in the following scheme.

The result of the enzymatic reaction of SOX is the intermediate glucose, which is cariogenic, but has a short life-span as it is fast converted to gluconic acid. A further advantage of the present invention is that only one mole of gluconic acid is formed for every two moles of hydrogen peroxide.

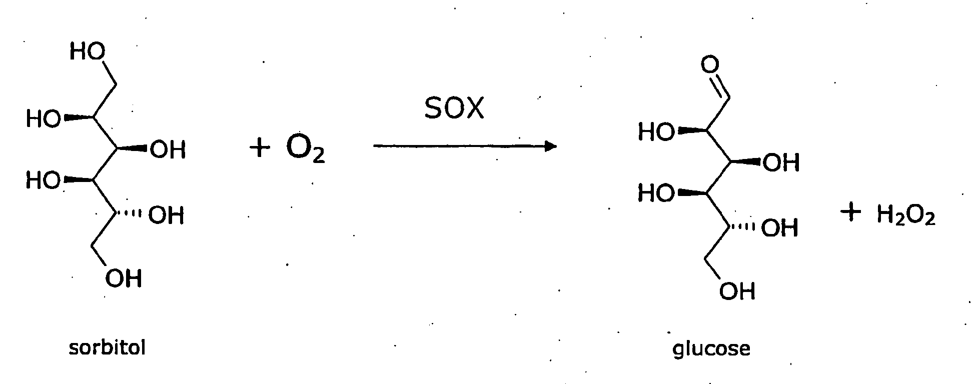

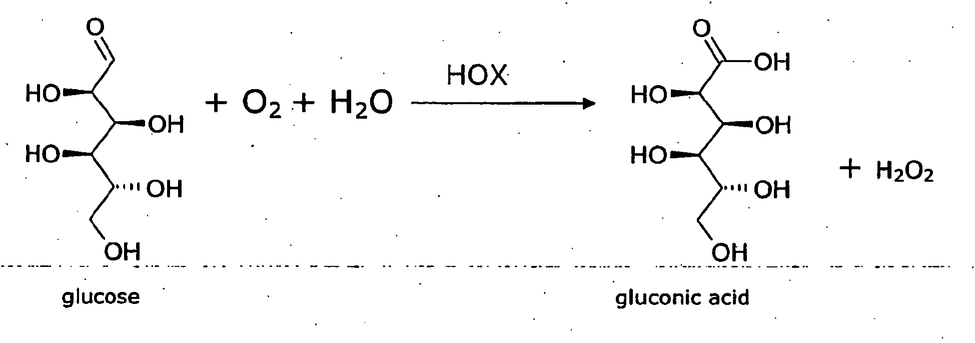

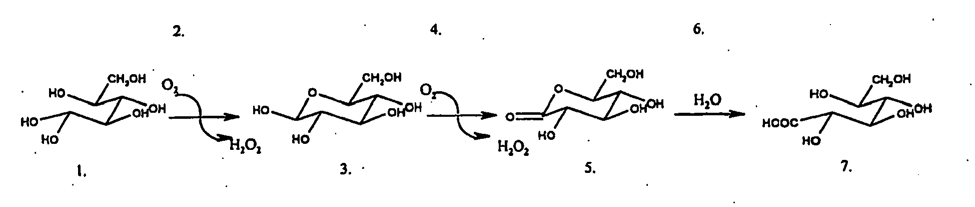

The reaction presented above represents the conversion of a polyol to the corresponding acid, by two consecutive enzymatic steps. Exemplified is the conversion of D-sorbitol to gluconic acid. (1) D-sorbitol, (2) Catalysis by polyol oxidase (3) D-glucose (4) Catalysis by hexose oxidase, glucose oxidase, glucooligosaccharide oxidase, carbohydrate oxidase (5) D-glucono-1,5-lactone (6) Hydrolysis in aqueous environment (7) D-gluconic acid.

The reaction may be generalised as:

| | First Oxidase | | Further Oxidoreductase | |
|---|---|---|---|---|
| Polyol | → | sugar | → | lactone |

Further specific examples include:

| | | | | |
|---|---|---|---|---|
| Sorbitol | → | glucose | → | D-glucono-1,5-lactone |
| Galactitol | → | galactose | → | D-galactono-1,5-lactone* |
| Mannitol | → | Mannose | → | D-mannono-1,5-lactone |
| Lactitol | → | lactose | → | D-lactonono-1,5-lactone |
| Xylitol | → | xylose | → | D-xylono-1,5-lactone |

| | | | | |
|---|---|---|---|---|
| *(or D-galactohexadialdose (if galactose oxidase is used) oxidises at C6) | | | | |

The lactone product is typically converted to an acid in an aqueous environment. The first oxidase may be selected from those disclosed herein. The further oxidoreductase enzyme is a sugar oxidase as refered to herein, such as Hexose oxidase or mannose oxidase (for mannose).

The amount of each enzyme present in the composition of the invention, or the total amount of enzyme present, or added to the composition or (application) products as referred to herein will depend on the enzymes used and the desired formulation required, but typically may range from about 0.0001% to about 20%, such as about 0.001% to about 10%, such as about 0.005% to about 2%, such as about 0.01% to about 1% by weight of the final composition,

Typically, the coupling of the two enzymes has been found to give proximately 200-300% the rate of production of hydrogen peroxide compared to a first oxidase first substrate system alone. Although considerable improvements in hydrogen peroxide were obtained using an equivalent unit to unit dose of both the polyol oxidase (such as sorbitol oxidase), and the further oxidoreductase such as hexose or glucose oxidase, the synergy was further enhanced by adding an excess of the further oxidoreductase to the composition, resulting In more hydrogen peroxide produced and at a faster rate.

In one aspect of the invention the composition comprises a sorbitol oxidase and a hexose oxidase and as the first substrate D-sorbitol.

In another aspect of the invention the composition comprises a sorbitol oxidase and a glucose oxidase and as the first substrate D-sorbitol.

In one embodiment, such as when the further oxidoreducatase is HOX, the polyol oxidase, such as sorbitol or xylitol oxidase may catalyse the oxidation of D-xylitol to yield 1 mole of xylose and 1 mole of H₂O₂. This may be advantageous particularly in embodiment where there is an alternative polyol other than xylitol, such as sorbitol, in that due to the low activity of HOX on xylose, xylose will accumulate. Xylose is a prebiotic compound. Hence such compositions can be used in food and feed compositions, or in a medicament, or in the preparation of food or feed composition or medicament, to enhance the xylose content, whilst also obtaining the other beneficial effects of the invention.

The use of polyol oxidase, such as sorbitol oxidase or xylitol oxidase, and a polyol, such as D-xylitol or sorbitol, has the advantage that an anti-microbial and/or whitening effect by H₂O₂ is obtained using non-toxic ingredients, and the addition of cariogenic compounds such as sugar is avoided.

### The Composition Matrix

The composition according to the invention may, suitably, comprise other materials typically used in the products and applications referred to herein, or other suitable applications/products.

The matrix materials are suitably selected to ensure compatability with the enzymes used in the composition to allow an effective amount of the enzymes to be used.

### The Product

As described above, a preferred embodiment is when the action of the further oxidoreductase on the second substrate generates further hydrogen peroxide and a product. The product is therefore other than hydrogen peroxide.

In one embodiment, the product obtained by the action of the further oxidoreductase on the second substrate may be more than a single product, *i.e*. may be products.

The accumulation of the product in the composition of the invention, or In the applications described herein, preferably does not negatively affect the production rate of hydrogen peroxide from the conversion of the first substrate by the first oxidase. Indeed, as the present inventors have discovered, the conversion of the second substrate to the product effectively removes the second substrate which not only greatly enhances the rate of generation of hydrogen peroxide by affecting the activity of the first oxidase on the first substrate, but also reduces the accumulation of possible undesirable second substrate.

In a preferred embodiment, the product is produced by the oxidation of the second substrate by the further oxidoreductase, and may for example be a lactone (oxidation at C1), a dialdose (oxidation at C5 for pentoses, C6 for hexose etc). One example is D-galactohexadialdose produced by galactose oxidase or a dehydro-sugar if oxidation occurs at any other position in the middle of the sugar chain. A further example is 2-dehydro-D-glucose produced by pyranose oxidase by oxidation of glucose at C2.

The product may be selected from the group consisting of D-glucono-1,5-lactone, D-xylono-1,5-lactone, D-maltono-1,5-lactone, D-mannono-1,5-lactone, D-galactono-1,5-lactone, D-hexadialdose, D-lactono-1,5-lactone, D-arabono-1,5-lactone, D-erythrono-1,5-lactone, D-ribono-1,5-lactone, D-lyxona-1,5-lactone, D-allono-1,5-lactone, D-altrono-1,5-lactone, D-gulono-1,5-lactone, D-idono-1,5-lactone, D-talono-1,5-lactone, and the lactone of isomaltutose, and possibly some more products from the oxidation of galactose oxidase on the C6 position of sugars.

### Definitions

Unless otherwise Indicated, the practice of the present invention involves conventional techniques commonly used In molecular biology, microbiology, protein purification, protein engineering, protein and DNA sequencing, recombinant DNA fields, and industrial enzyme use and development, all of which are within the skill of the art.

Unless defined otherwise herein, all technical and scientific terms used herein have the same meaning as commonly understood by one of ordinary skill in the art to which this invention pertains: For example, Singleton and Sainsbury, Dictionary of Microbiology and Molecular Biology, 2d Ed., John Wiley and Sons, NY (1994); and Hale and Margham, The Harper Collins Dictionary of Biology, Harper Perennial, NY (1991) provide those of skill in the art with a general dictionaries of many of the terms used in the invention. Although any methods and materials similar or equivalent to those described herein find use in the practice of the present invention, preferred methods and materials are described herein. Accordingly, the terms defined immediately below are more fully described by reference to the Specification as a whole. Also, as used herein, the singular terms "a," "an," and "the" include the plural reference unless the context clearly indicates otherwise. Unless otherwise indicated, nucleic acids are written left to right in 5' to 3' orientation; amino acid sequences are written left to right in amino to carboxy orientation, respectively. It is to be understood that this invention is not limited to the particular methodology, protocols, and reagents described, as these may vary, depending upon the context they are used by those of skill in the art.

It is intended that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

When referring to a "respective polyol substrate" of an enzyme, it refers to the substrate which the enzyme utilises, e.g. the respective substrate of sorbitol oxidase is sorbitol, and the respective substrate of xylitol oxidase is xylitol.

As used herein, the term "compatible," means that the composition matrix materials (other ingredients) do not reduce the enzymatic activity of the oxidase enzyme(s) provided herein to such an extent that the oxidases(s) is/are not effective as desired during normal use situations. Specific cleaning composition materials are exemplified in detail hereinafter.

As used herein, "effective amount of enzyme" refers to the quantity of enzyme necessary to achieve the enzymatic activity required in the specific application. Such effective amounts are readily ascertained by one of ordinary skill in the art and are based on many factors, such as the particular enzyme variant used, the cleaning application, the specific composition of the cleaning composition, and whether a liquid or dry (e.g., granular) composition is required, and the like.

Amino acid and polynucleotide homology may be determined using ClustalW algorithm using standard settings: see http://www,ebi,ac,uk/emboss/align/index,html, Method : EMBOSS::water (local): Gap Open = 10.0, Gap extend = 0.5, using Blosum 62 (protein), or DNAfull for nucleotide sequences.

It is preferable the when referring to "the second substrate is convertable by the at least one further oxidoreductase to form a product", that the second substrate is oxidisable by the further oxidoreductase to form hydrogen peroxide and a (the) product.

The term "variant(s)" as used herein In the context of a polypeptide (sequence), such as SEQ ID NO 1 and SEQ ID NO 2 refers to a polypeptide which is prepared from the original (parent) polypeptide, or using the sequence Information from the polypeptide, by insertion, deletion or substitution of one or more amino acids in said sequence, i.e. at least one amino acids, but preferably less than about 50 amino acids, such as less than about 40, less than about 30, less than about 20, or less than about 10 amino acids, such as 1 amino acid, 1-2 amino acids, 1-3 amino acids, 1-4 amino acids, 1-5 amino acids.

The term "homologue(s)" as used herein in the context of a polypeptide sequence, such as a SEQ ID NO 1 and SEQ ID NO 2 refers to a polypeptide which is at least about 70% homologous, such as at least about 80% homologous, such as at least about 85% homologous, or at least about 90% homologous, such as at least about 95%, about 96%, about 97%, about 98% or about 99% homologous to said polypeptide sequence. Homology between two polypeptide sequences may be determined using ClustalW alignment algorithm using standard settings, as referred to herein.

The term "fragment(s)" as used herein in the context of a polypeptide sequence, such as a SEQ ID NO 1 and SEQ ID NO 2 refers to a polypeptide which consists of only a part of the polypeptide sequence. A fragment may therefore comprise or consist of at least about 50%, such as at least about 60%, such as at least about 70%, such as at least about 80%, such as at least about 90% or such as at least about 95% of said polypeptide sequence.

The variant, homologue and fragment according to the invention all retain at least part of the desired (for the purose of the present invention) enzymatic activity of the parent enzyme, such as at least about 10%, at least about 20%, at least about 30%, at least about 40%, at least about 50%, at least about 60%, at least about 70%, at least about 80% at least about 90% or all the enxyme activity of the parent enzyme.

It will be recognised by the skilled person that when we identify preferred enzymes for use In the composition and methods of the invention by their specific SEQ IDs, this includes enzymes which are derived from the nucleic acids which encode the corresponding amino acid SEQ IDs when expressed, either in their native host species or a heterologous host species, and as such the enzymes may be co- or post- translationally processed.

The term "about" as used herein to refer to concentrations, activities and conditions etc. includes and specifically discloses the exact values and exact ranges referred to.

The term "polyol" as used herein refers to a sugar alcohol which comprises more than one hydroxyl group. Polyol is a distinct term from sugar as polyols only contain hydroxyl (COH) groups and belong to the general group alditols, whilst sugars have carbonyl groups (COOH).

Disaccharides arid monosaccharides can both form sugar alcohols; however, sugar alcohols derived from disaccharides (eg. Maltitol and lactitol) are not entirely hydrogenated because only one aldehyde group is available for reduction. In one embodiment the sugar alcohol are fully hydrogenated.

Sugar alcohols are commonly added to foods because of their lower caloric content than sugars; however they are also generally less sweet, and are often combined with high intensity sweeteners. They are also added to chewing gum because they are not metabolized (ie broken down) by bacteria in the mouth, so they do not contribute to tooth decay. Maltitol, sorbitol and Isomalt are some of the more common types. Sugar alcohols may be formed under mild reducing conditions from their analogue sugars.

As used herein, the term "oxidase" refers to enzymes that catalyze an oxidation/reduction reaction involving molecular oxygen (02) as the electron acceptor. In these reactions, oxygen is reduced to water (H₂O) or hydrogen peroxide (H₂O₂). The oxidases are a subclass of the oxidoreductases.

The term "polyol oxidase" refers to an enzyme which is capable of oxidizing a polyol to the corresponding sugar. The oxidation of the polyol by the action of the polyol oxidase results In the production of hydrogen peroxide.

As used herein, the term "glucose oxidase" ("Gox") refers to the oxidase enzyme (EC 1.1.3.4) that binds to beta-D-glucose (i.e., an isomer of the six carbon sugar, glucose) and aids In breaking the sugar down into its metabolites. GOx is a dimeric protein which catalyzes the oxidation of beta-D-glucose Into D-glucono-1,5-lactone, which then hydrolyzes to gluconic acid with concomitant reduction of molecular oxygen to hydrogen peroxide.

As used herein, the term "alcohol oxidase" ("Aox") refers to the oxidase enzyme (EC 1.1.3.13) that converts an alcohol to an aldehyde with concomitant reduction of molecular oxygen to hydrogen peroxide.

As used herein, the term "choline oxidase" ("Cox") refers to an oxidase enzyme (EC 1.1.3. 17) that catalyzes the four-electron oxidation of choline to glycine betaine, with betaine aldehyde as an intermediate with concomitant reduction of two molecules of molecular oxygen to two molecules of hydrogen peroxide.

As used herein, the term "hexose oxidase" ("Hox") refers to an oxidase enzyme (EC 1.1.3.5) the oxidation of mono- and disaccharides to their corresponding lactones, with concomitant reduction of molecular oxygen to hydrogen peroxide. Hexose oxidase is able to oxidize a variety of substrates including D-glucose, D-galactose, maltose, cellobiose, and lactose, etc.

As used herein, "glycerol oxidase" refers to an oxidase enzyme (EC 1.1.3. ) that catalyzes the oxidation of glycerol to glyceraldehyde, with concomitant reduction of molecularoxygen to hydrogen-peroxide.

It is recognised that the activity of an enzyme will depend on the conditions and substrates available, and therefore the activity of an enzyme may differ from a standard assay condition *(in vitro* assay), as compared to within a composition according the invention, or the use of such a composition in the desired application. In one embodiment, the enzyme activity is determined by an *in vitro* assay as referred to in the examples. Alternatively, the activity of an enzyme is determined *in situ, i.e,* in the composition according to the invention and under conditions which the composition is to be used. The same analytic methods may be used for determining the in situ enzyme activity as for the *in vitro* activity, just the assay is performed using the composition matrix, or under conditions in which the composition/product according to the invention are used.

The term "sugar" as used herein refers to monosaccharides, disaccharides and oligosaccharides, hexose, such as sugars selected from the group consisting of lactose, maltose, sorbose, triose, pentose, hexose, mannose, glucose, galactose, xylose, fructose, isomaltose, erythrose.

Sugars contain either aldehyde groups (-CHO) or ketone groups (C=O), where there are carbon-oxygen double bonds, making the sugars reactive. Preferably, the term sugar conforms to (CH2O)n where n is between 3 and 6, such as 3, 5 or 6, or 5 or 6.

The sugars may be trioses, pentose or hexose, preferably pentose or hexose sugars, preferably In closed-chain form.

The sugar may be selected from the group consisting of: sucrose, fructose, glucose, galactose, maltose, lactose and mannose.

By "oral care product" as used herein is meant a product which is not intentionally swallowed for purposes of systemic administration of therapeutic agents, but is retained in the oral cavity for a sufficient time to contact substantially all of the dental surfaces and/or oral mucosal tissues for purposes of oral activity. In the context of the present invention oral care product also includes products for cleaning dentures, artificial teeth and the like. The oral care product may have any suitable physical form such as e.g. powder, paste, gel, liquid, ointment, chewing gum tablet or spray.

As used herein, "cleaning compositions" and "cleaning formulations" refer to compositions that find use in the removal of undesired compounds from items to be cleaned, such as fabric, dishes, contact lenses, other solid substrates, hair (shampoos), skin (soaps and creams), teeth (mouthwashes, toothpastes) etc. The term encompasses any materials/compounds selected for the particular type of cleaning composition desired and the form of the product (*e.g*., liquid, gel, granule, or spray composition), as long as the composition is compatible with the oxidase and other enzyme(s) used in the composition, and any reversible enzyme inhibitors in the composition. The specific selection of cleaning composition materials are readily made by considering the surface, item or fabric to be cleaned, and the desired form of the composition for the cleaning conditions during use.

The terms further refer to any composition that is suited for cleaning, bleaching, disinfecting; and/or sterilizing any object and/or surface. It is intended that the terms include, but are not limited to detergent compositions (*e.g*., liquid and/or solid laundry detergents and fine fabric detergents; hard surface cleaning formulations, such as for glass, wood, ceramic and metal counter tops and window; carpet cleaners; oven cleaners; fabric fresheners; fabric softeners; and textile and laundry pre-spotters, as well as dish detergents).

Indeed, the term "cleaning composition" as used herein, includes unless otherwise Indicated, granular or powder-form all-purpose or heavy-duty washing agents, especially cleaning detergents; liquid, gel or paste-form all-purpose washing agents, especially the so-called heavy-duty liquid (HDL) types; liquid fine-fabric detergents; hand dishwashing agents or light duty dishwashing agents, especially those of the high-foaming type; machine dishwashing agents, including the various tablet, granular, liquid and rinse-aid types for household and institutional use; liquid cleaning and disinfecting agents, including antibacterial hand-wash types, cleaning bars, mouthwashes, denture cleaners, car or carpet shampoos, bathroom cleaners; hair shampoos and hair-rinses; shower gels and foam baths and metal cleaners; as well as cleaning auxiliaries such as bleach additives and "stain-stick" or pre-treat types.

As used herein, the terms "detergent composition" and "detergent formulation" are used in reference to mixtures which are intended for use in a wash medium for the cleaning of soiled objects. In some preferred embodiments, the term is used in reference to laundering fabrics and/or garments (*e.g*., "laundry detergents"). In alternative embodiments, the term refers to other detergents, such as those used to clean dishes, cutlery, etc. (*e.g*., "dishwashing detergents"). It is not intended that the present invention be limited to any particular detergent formulation or composition. Indeed, it is intended that in addition to oxidase, the term encompasses detergents that contain surfactants, transferase(s), hydrolytic enzymes, oxido reductases, ,perhydrolases builders, bleaching agents, bleach activators, bluing agents and fluorescent dyes, caking inhibitors, masking agents, enzyme activators, enzyme inhibitors, antioxidants, and solubilizers. In some preferred embodiments, the detergent formulations include, but are not limited to those set forth in US Pat. Appln. Ser. Nos. 10/576,331 and 10/581,014, as well as WO 05/52161 and WO 05/056782 find use in the present invention. However, it is not intended that the present invention be limited to any particular detergent formulation(s), as any suitable detergent formulation finds use in the present invention.

As used herein, "dishwashing composition" refers to all forms of compositions for cleaning dishware, including cutlery, including but not limited to granular and liquid forms. It is not intended that the present invention be limited to any particular type or dishware composition. Indeed, the present invention finds use in cleaning dishware (*e.g*., dishes, including, but not limited to plates, cups, glasses, bowls, etc.) and cutlery (*e.g*., utensils, including but not limited to spoons, knives, forks, serving utensils, etc.) of any material, including but not limited to ceramics, plastics, metals, china, glass, acrylics, etc. The term "dishware" is used herein in reference to both dishes and cutlery.

As used herein, "wash performance" of an enzyme refers to the contribution of an enzyme to washing that provides additional cleaning performance to the detergent without the addition of the enzyme to the composition. Wash performance is compared under relevant washing conditions.

The term "relevant washing conditions" is used herein to indicate the conditions, particularly washing temperature, time, washing mechanics, sud concentration, type of detergent and water hardness, actually used in households in a detergent market segment.

The term "Improved wash performance" is used to indicate that a better end result Is obtained in stain removal from items washed (e.g., fabrics or dishware and/or cutlery) under relevant washing conditions, or that less enzyme, on weight basis, is needed to obtain the same end result relative to another enzyme.

The term "retained wash performance" is used to indicate that the wash performance of an enzyme, on weight basis, is at least 80% relative to another enzyme under relevant washing conditions.

Wash performance of enzymes is conveniently measured by their ability to remove certain representative stains under appropriate test conditions. In these test systems, other relevant factors, such as detergent composition, sud concentration, water hardness, washing mechanics, time, pH, and/or temperature, can be controlled in such a way that conditions typical for household application in a certain market segment are imitated.

As used herein, the term "disinfecting" refers to the removal of contaminants from the surfaces, as well as the inhibition or killing of microbes on the surfaces of items. It is not intended that the present invention be limited to any particular surface, item, or contaminant(s) or microbes to be removed.

### Control of the reaction

Both oxidation reactions require a presence of oxygen and would thus not take place to a significant degree as long as the composition is contained in a sealed container, such as in a controlled oxygen environment (oxygen limited environment), such as in the exclusion of limitation or absence of molecular oxygen. Once the container is open and the composition is subjected to atmospheric air or alternative source of molecular oxygen, the reaction can take place.

The invention therefore also provides for a packaged product comprising the composition of the invention, wherein the composition is maintained in a controlled oxygen environment, or a oxygen limited environment, such as in the absence of (available) molecular oxygen, so as to prevent or reduce the production of hydrogen peroxide within said packaged product.

An alternative method is providing the compositon of the invention in a controlled water environment, where the level of water is sufficiently low to prevent or reduce the production of hydrogen peroxide within the composition or a packaged product. Suitably the level of water in such a composition may be less than about 2%, such as less than about 1%, such as less than about .5%, such as less than about 0.2% or less than about 0.1%.

Alternatively the reaction may be prevented from occurring prematurely by physically separating the enzyme and substrate components from each other by compartmentalisation methods well known in the art. In one embodiment, the reaction is prevented by separating the first substrate from the composition compartment comprising the first oxidase and the further oxidoreductase. The first substrate may be added to activate the composition, or may form part of the application matrix, either as a routine ingredient of said application matrix, or supplemented to said application matrix either before, during or prior to the addition of the composition. The invention therefore also provides for a first composition comprising the first oxidase (such as polyol oxidase) and the further oxidoreductase, as referred to herein, in a kit of parts, with a further composition comprising said first substrate, wherein said first and second compositions are separated from one another.

Although it is envisaged that the composition of the invention may be provided as a two or more pot system, for combinantion upon use, it is also considered that technologies such as encapsulation or micro-encapsulation may be employed to keep the enzyme component of the composition separated from the substrates. The release from the micro-encapsules may be triggered by, for example mechanical force or dilution upon use of the composition of the invention.

In one aspect the compositon according to the invention comprises a kit of parts comprising at least two pots, wherein the first pot comprises the first oxydase and the further oxidoreductase, and a second pot which comprises the first substrate.

In the applications disclosed herein, such as in cosmetic applications (also in oral care application), in one embodiment it is advantageous to compartmentallse the composition of the invention, so that the production of hydrogen peroxide is achieved upon application, for example by mechanical scrubbing allowing the release of the enzyme system and letting it come into contact with first substrate *in situ* during application, but not during storage.

In one aspect of the invention the pH of the composition is between 5 and 9, preferably between 6 and 8. When the composition according to the invention is an oral care product it is preferred to use a first oxidase, such as a polyol oxidase, such as SOX and an oxidoreductase, which is substantially active at the pH prevailing in the mouth *i.e*. at a pH between (about) 5 and (about) 9, preferably between about 6 and about 8. It is furthermore preferred that the first oxidase, such as polyol oxidase, such as sorbitol oxidase and the oxidoreductase are active at ambient temperature, or at body temperature.

In one embodiment the first oxidase, and or further oxidoreductase, and any other enzymes present may be immobilized.

### Further Enzymes and enzyme systems

The composition according to the invention may comprise further enzymes such as one or more enzymes selected from the group consisting of: peroxidases such as lactoperoxidase, laccases, amyloglucosidase, amylases such as maltogenic and non-maltogenic amylases, such as Novamyl^{™}, glucoamlyase, dextranase, protease, lysozyme, mutanase, lipase, acyl-transferase, such as the acyl-transferases disclosed in WO2004064537, and xylanase.

In one embodiment, the composition of the invention, such as the oral compositon of the invention, or alternative compostions as referred to herein, comprise thiocyanate, thereby allowing the conversion of thiocyanate to hypothiocyanate due to the production of hydrogen peroxide. For oral care products, there is, typically, sufficient lactoperoxidase present in the saliva in the mouth to convert thiocyanate to hypothiocyanate in the presence of hydrogen peroxide. However, the composition according to the invention may also comprise lactoperoxidase, to facilitate this conversion, thereby allowing the production of an effective antibacterial agent *in situ.*

It is recognized that the rate of the release of hydrogen peroxide may become limited if the availability of oxygen is limiting. It is therefore considered that coupling of the present enzyme system to a oxygen generation system may be appropriate.

### Applications

The composition according to the invention can be used to treat any type of material where whitening and/or bleaching is desired such as e.g. teeth, paper and textiles.

An advantage of the use of this combination of enzymes for bleaching and/or whitening is that the whitening and/or bleaching effect is obtained by the use of non-hazardous materials compared to previous use of bleaching agents in e.g. oral care products.

The composition according to the invention has furthermore an anti-microbial effect due to the resulting hydrogen peroxide. A further aspect of the invention relates to the use of the composition according to the invention in the manufacture of an oral care product for the treatment or prevention of microbial effects relating to breath malodour or periodontitis.

Other beneficial uses of the present composition are in food preservation where the composition acts as an oxygen scavenger using two moles of O₂ every time one mole of sugar is used.

In the personal care area, the composition of the present invention may be added to toothpaste, in particular, whitening teeth, mouthwash, denture cleaner, liquid soap, skin care creams and lotions, hair care and body care formulations and solutions for cleaning contact lenses in an amount effective to act as an antibacterial agent. The composition of the present invention may also be a component of a laundry detergent composition or a dishwashing detergent composition and may be used as a hydrogen peroxide source. The laundry detergent composition may comprise a surfactant, said the composition of the present invention. The dishwashing detergent composition may comprise said the composition of the present invention and a bleach precursor or peroxy acid. The composition of the present Invention may particularly be useful for removing stains.

### Detergents

The detergent (product) according to the invention comprises the composition according to the invention. Therefore, in one embodiment the invention provides for the use of the composition of the invention in a detergent product, such as a cleaning composition, cleaning formulation, detergent composition or detergent formulation.

The detergent (product) may be used as a bleaching or whitening agent, or may be used as a disinfecting agent, or both bleaching/whitening and disinfecting agent.

The detergent (product) may comprise further enzymes or enzyme systems, as disclosed herein in, in particular peroxidases, proteases, amylases, lipases, acyl-transferases and lactoperoxidases (optionally In the presence of thiocyanate).

In one embodiment, the detergent product may be in the form of a cleaning composition or cleaning formulation.

In one embodiment, the detergent product may be in the form of a detergent composition or detergent formulation.

The detergent product may be a dishwashing composition.

Preferably the detergent product according to the invention has an improved wash performance, under the relevant washing conditions, as compared to an equivalent product which does not comprise the composition of the invention.

In some embodiments, detergent formulations comprising the composition of the invention, and a bleach booster are used to produce active oxygen species in laundry wash liquor to bleach stains.

In some embodiments, the detergent composition further comprises an acyl-transferase and its substrate are used to produce active oxygen species in laundry wash liquor to bleach stains.

In a one aspect the composition is an edible composition, such as an oral care composition, an (orally administered) medicament composition, or a food or feed composition.

The term 'edible composition' refers to compositions which are for oral administration or for consumption via the oral cavity.

The edile compositions may comprise one or more of flavourings, preservatives, textural ingredients, emulsifiers, sweeteners, humectants, and/or binding agents which (typically) have been approved for human (or animal) consumption.

### Oral Care

In one aspect of the Invention an oral care product comprising a composition according to the invention and ingredients used in oral care products, is provided.

In a further aspect of the invention the oral care product according to the invention comprises a first oxidase, such as polyol oxidase, such as sorbitol oxidase, a further oxidoreductase and a polyol, such as sorbitol and ingredients used In oral care products where antimicrobial effect and/or whitening and/or bleaching is desired, is provided.

Examples of oral care products include toothpaste, dental cream, gel or tooth powder, odontic, mouth rinses, mouth sprays, pre- or post brushing rinse formulations, chewing gum, lozenges, and candy.

The oral care product may comprise further active Ingredients such as thiocyanate, zinc gluconate, lysozyme, lactoferrin, lactoperoxidase, and amyloglucosidase, Further ingredients are listed in WO97/06775, and include redox mediators.

In one embodiment the oral care composition/product according to the invention further comprises fluoride.

Toothpastes and tooth gels typically include ingredients such as abrasive polishing materials, foaming agents, flavouring agents, humectants, binders, thickeners, sweetening agents, whitening/bleaching/stain removing agents, water, and optionally enzymes.

Mouth washes, including plaque removing liquids, typically comprise ingredients such as a water/alcohol solution, flavour, humectant, sweetener, foaming agent, colorant, and optionally enzymes.

Chewing gum: Suitable compositions for the preparation of a chewing gum are disclosed in WO2005/006872 and US 4,564,519.

When used in oral care products the amount of first oxidase, such as polyol oxidase, such as sorbitol oxidase, and further oxidoreductase should be a safe and effective amount which means an amount high enough to significantly modify the condition to be treated or effect the desired whitening and/or bleaching result but low enough to avoid serious side effects.

In a further aspect the invention provides a method for bleaching and/or whitening of teeth, comprising contacting the teeth with an oral care product comprising a composition according to the Invention in an amount and time suitable for bleaching and/or whitening teeth.

The present invention provides a safe teeth whitening composition that has the advantage over the prior art by providing use of a first oxidase, such as a polyol oxidase, such as sorbitol oxidase which can act on substrates which are non-cariogenic (*i.e*. substrates which do not degrade into cariogenic sugars such as sucrose, glucose, fructose, maltose etc.).

### Food and Feed

The composition of the invention may be used in general disinfecting of food and feed and food and feed environments, such as manufacturing, processing an preparation facilities, such as milking parlours, cheese making facilities, meat processing factories, vegetable and fruit washing and processing plants, in restaurants etc.

In meat (*i.e*. animal meat, including fish) processing factories, the composition of the invention may be used In washing and disinfecting animal carcasses and food products derived therefrom. The use of the polyol oxidase/further oxidoreductase system is particularly preferred as this reduces the level of fermentable sugars present in or on the surface of the meat products, reducing the growth of undesirable micro-organisms. The composition according to the invention may further comprise alkali silicates (US2004062851, hereby incorporated by reference), such compositions are considered particularly useful in decontaminating meat products. The use of alakali silicates also Increased the water retention properties of meant and meat products, and can be used to enhance the retention of the composition of the invention in meat and meat products.

A further advantage Is the lowering of the pH due to the generation of acidic further products, which also inhibits the generation of micro-organisms in or on the surface of the meat. Similar benefits are seen in vegetable and fruit washing and processing facilities.

The compostion of the invention may also be used in food and feed products. Hydrogen peroxide is used extensively as an anti-microbial agent In the production of darly products and egg products. Suitably, the composition according to the invention may be added to, or may be a food product selected from the group consisting of: dairy products, such as milk, cream, cheese, whey, yoghurt, butter, or egg, such as egg yolk or egg white.

The lowering of the pH due to the accumulation of acidic further products is also a considerable advantage in other food types, such as in cheese production, where the rapid lowering of the pH increases the rate of maturation of cheese, such as cheddar cheeses and Italian hard cheeses, reducing the maturation and storage time. The lower pH therefore also contributes to an enhanced taste of cheese.

In one embodiment the composition of the invention is used in a beverage, such as a fruit juice. Fruit juices typically comprise suitable first substrates, therefore it may be unnecessary to add further first substrate to the fruit juice or alternatively they may be supplemented with the first substrates referred to herein.

Furthermore. O₂ is scavenged by the production of xylose, which can prevent food spoilage. A further benefit is the lowering of pH compared to a system where SOX is used alone, where no such lowering of pH is observed - the lowering of pH can also prevent or reduce food spoilage.

### Personal Care products

In the personal care area, the composition of the present invention may be added to a personal care product other than oral care products, such as denture cleaner, liquid soap, skin care creams and lotions, hair care and body care formulations and solutions for cleaning contact lenses in an amount effective to act as an antibacterial agent.

### Paper production

The composition of the invention may also be used for cleaning paper mills, the hydrogen peroxide produced oxidizes carbohydrates to the corresponding acids, which in turn are believed to chelate with the cationic part of inorganic salts such as scale. This enables better cleaning and/or control of sediments, and for water streams it leads to a turbidity reduction, to an improved settling behaviour, as well as to a colour reduction, all of which makes cleaning and effluent control procedures easier and less expensive (see WO06/061018).

### Cosmetics

Glucose oxidase has been used as the basis of a commercial preservative system for cosmetics and toiletries. However, the present invention provides for a suitable replacement for the use of glucose oxidase as it provides a far more effective system for producing hydrogen peroxide, and/or may also provide an effective anti- microbial/anti-bacterial action when in use. It may also provide a dual function product, being a cosmetic which also bleaches or whitens the skin during use.

The lactoperoxidase system is used in numerous cosmentic products. The cosmetic composition or product according to the invention may therefore further comprise lactoperoxidase and thiocyanate.

Polyols, such as sorbitol is typically a major component of cosmetics, or may be added to cosmetics to provide a suitable substrate for the polyol oxidase in the composition according to the invention.

The use of the composition of the invention in cosmetic products may provide one or more of the following benefits: preservative of the cosmetic, and microbial/bacterial activity when applied to the skin, skin lightening/bleaching effect.

Cosmetic ingredients include: Antioxidants, binding agents, emollients, emulsifiers, humectants, pigments, lubricants, preservatives, solvents, fragrances, surfactants, vehicle substances, such as an inert base material, stabilizers and thickeners.

The composition/product of the invention may be used in the form of one or more of the cosmetic products selected form the group consisting of: Lipstick, lip gloss, lip pencil, and Lip-Ink; liquid foundation; Cream foundation; Powder; Rouge (blush or blusher); bronzer; Mascara; Eyeliner and eye shadow; Nail polish; Concealer; skin care products such as creams and lotions, e.g. to moisturize the face and body; sunscreens and sun lotions; treatment products to repair or hide skin imperfections;

Cosmetics can also be described by the form of the product, as well as the area for application. Cosmetics can be liquid or cream emulsions; powders, both pressed and loose; dispersions; and anhydrous creams or sticks.

The composition/products according to the invention may also be used in cosmetic techniques such as skin beaching and/or whitening.

### Skin beaching

Hydroquinone has been used as an ingredient in preparations for skin beaching. However, recently the US Environmental Protection Agency has issued a notice of proposed rulemaking that would establish that over-the-counter (OTC) skin bleaching drug products based on hydroquinone are potentially carcinogenic and not generally recognized as safe and effective (GRAS) and are misbranded.

The compositions and products according to the invention may be used for the preparation of enzyme based products for skin beaching, and therefore provide a safe alternative to the use of hydroquinone.

Skin bleaching products may comprise other bleaching agents, such as licorice extract, mulberry extract, arbutin, kojic acid, bearberry extract, AHA blends, salicylic acids, aloeleic acid, citric acids, lactic acids, as well as suitable base matrixies, and sunscreens.

### Hair bleaching

For hair colouring bleaching a chemical oxidizing agent is used. Suitable oxidizing agents are persulfates, chlorites and, above all, hydrogen peroxide or addition products thereof with urea, melamine and sodium borate. Therefore the composition of the present invention may also be used as a safe alternative source of hydrogen peroxide for hair bleach.

Suitably a hair beaching composition may also be used for colouring keratin fibers, and may therefore contain at least one dye precursor as well as the composition of the present invention. The present invention also relates to a process for coloring keratin containing fibers using the composition of the present invention.

### Paint

The term 'paint' as used in the context of the present invention herein refers to the family of products used to protect and/or add color to an object or surface by covering it with a pigmented or non-pigmented coating, and includes varnish, wood stains, shellac, lacquer, and enamel. Paint can be applied to almost any kind of object. Paint is a semifinished product, as the final product is the painted article itself.

The paint may be for used in application where the dried paint is exposed to a natural water environment, such as a maritime paint. Maritime paints are used to prevent or reduce anti-fouling on the hulls of boats and ships caused by the growth of organisms such as algar, barnicles and the like.

The paint may be a decorative paint, for example used in the interior or exterior of buildings and other objects.

The paint may be a protective paint, preventing or reducing microbial spoilage of the surface or material upon which the paint is applied, such as wet rot or dry rot.

The compostion according to the invention can be used as a preservative in paint. Alternatively or in addition, the composition can be used in a paint for reducing fouling, such as in maritime paint, where the production of hydrogen peroxide causes an oxidative layer that prevent or reduces the attachment or growth of fouling organisms on the surface. Such paints which comprise the composition according to the invention offer a beneficial enzyme solution to this problem as the hydrogen peroxide is produced without the addition, or significant accumulation of fermentable substrates within the paint, which can actually encourage anti-fouling, especially if the enzymes within the paint become Inactivated.

### Pesticides

Pesticide products usually contain no more than 35% hydrogen peroxide, which Is then usually diluted to 1% or less when applied as a spray or a liquid. Hydrogen peroxide is used for many non-food and food crops (e.g., fruits, nuts, and vegetables), both indoors and outdoors, and before and after harvest and for disinfecting food storage facilities. The target Pests are typically microbes, including fungi and bacteria which cause plant diseases. The hydrogen peroxide containing pesticides, used to prevent and control plant pathogens, are typically applied as a spray on foliage, or as a dip on cuttings and roots, or as a pre-planting soil treatment.

The composition according to the invention may therefore be used as a pesticide, either directly, allowing production of hydrogen peroxide *in situ,* or as a system for generation of hydrogen peroxide which may subsequently be applied to the appropriate surface.

Further disclosed herein:
1. A composition comprising
   a sorbitol oxidase, a first substrate, and an oxidoreductase,
   wherein the first substrate is oxidisable by the sorbitol oxidase to form hydrogen peroxide and a second substrate, and the second substrate is oxidisable by the oxidoreductase to form hydrogen peroxide and a product.
2. The composition according to the invention such as to embodiment 1, wherein the first substrate is one or more selected from the group consisting of D-sorbitol, D-xylitol, D-mannitol, D-arabitol, glycerol, inositol, 1,3-propanediol, 1,3-butanediol, and 1,4-butanediol.
3. The composition according to the invention such as to embodiment 2, wherein the first substrate is one or more selected from the group consisting of D-sorbitol or D-xylitol.
4. The composition according to the invention such as to embodiment 3, wherein the first substrate is D-sorbitol.
5. The composition according to the invention such as to embodiment 1, wherein the oxidoreductase is one or more selected from the group consisting of hexose oxidase, glucose oxidase, carbohydrate oxidase, and oligosaccharide oxidase.
6. The composition according to the invention such as to embodiment 5, wherein the oxidoreductase is one or more selected from the group consisting of a carbohydrate oxidase, a hexose oxidase or a glucose oxidase.
7. The composition according to the invention such as to embodiment 6, wherein the oxidoreductase is a hexose oxidase.
8. The composition according to the invention such as to embodiment 1, wherein the sorbitol oxidase is derived from a strain of Streptomyces.
9. The composition according to the invention such as to any one of the embodiments 1-8, wherein the pH of the composition is between 5 and 9.
10. The composition according to the invention such as to any one of the embodiments 1-9, wherein the pH of the composition is between 6 and 8.
11. The composition according to the invention such as to any one of the embodiments 1-10, wherein the sorbitol oxidase and the oxidoreductase are active at ambient temperature.
12. An oral care product comprising a composition according to the invention such as to any one of embodiments 1-11 and ingredients used in oral care products.
13. The use of a sorbitol oxidase for whitening and/or bleaching.
14. The use of a composition according to the invention such as to any one of the embodiments 1-10 for whitening and/or bleaching.
15. The use according to the invention such as to any one of the embodiment 13-14 for whitening and/or bleaching teeth.
16. A method for bleaching and/or whitening of teeth, comprising contacting the teeth with an oral care product comprising a composition according to the invention such as to any one of the embodiments 1-10 in an amount and time suitable for bleaching and/or whitening teeth.
17. Sorbitol oxidase and D-xylitol for use as a medicament.
18. A medicament according to the invention such as to embodiment 17, wherein the sorbitol oxidase is derived from a strain of Streptomyces.
19. An oral care product comprising sorbitol oxidase and D-xylitol, and ingredients used in oral care products.
20. The use of a sorbitol oxidase and D-xylitol for whitening and/or bleaching of teeth.

### EXAMPLES

The following examples are provided in order to demonstrate and further illustrate certain preferred embodiments and aspects of the present invention and are not to be construed as limiting the scope thereof.

In the experimental disclosure which follows, the following abbreviations apply: °C (degrees Centigrade); rpm (revolutions per minute); H₂O (water); HCl (hydrochloric acrid); aa (amino acid); bp (base pair); kb (kilobase pair); kD (kilodaltons); gm (grams); µg and ug (micrograms); mg (milligrams); ng (nanograms); µl and ul (microliters); ml (milliliters); mm (millimeters); nm (nanometers); µm and um (micrometer); M (molar); mM (millimolar); µM and uM (micromolar); U (units); V (volts); MW (molecular weight); sec (seconds); min(s) (minute/minutes); hr(s) (hour/hours); MgCl₂ (magnesium chloride); NaCl (sodium chloride); OD₂₈₀ (optical density at 280 nm); OD₆₀₀ (optical density at 600 nm); EFT ("effective fermentation time"); HDL (Heavy Duty Detergent Liquid ); EtOH (ethanol); PBS (phosphate buffered saline [150 mM NaCl, 10 mM sodium phosphate buffer, pH 7.2]); SDS (sodium dodecyl sulfate); Tris (tris(hydroxymethyl)aminomethane); TAED (N,N,N'N'-tetraacetylethylenediamine); w/v (weight to volume); v/v (volume to volume); GOX and GOx (glucose oxidase); AOX and AOx (alcohol oxidase); COX and Cox (choline oxidase); HOX and HOx (hexose oxidase); SOX and Sox (sorbitol oxidase); AATCC (American Association of Textile and Coloring Chemists); WFK (wfk Testgewebe GmbH, Bruggen-Bracht, Germany); Testfabrics (Testfabrics Inc, Pittston PA,); ATCC (American Type Culture Collection, Manassas, VA); Geneart (Geneart, Regesburg, Germany); Invitrogen (Invitrogen, Inc., Carlsbad, CA); Baker (J.T.Baker, Phillipsburg, NJ); NAEF (NAEF, Press and Dies, Inc., Bolton Landing, NY); Fluka (Fluka Chemie AG, Buchs, Switzerland); Prometric (Prometic Biosciences, Wayne NJ); Minolta (Konica Minolta. Glen Cove, NY); and Sigma (Sigma-Aldrich Chemical Co., St. Louis, MO).

### EXAMPLE 1: Construction of Strains Expressing Sorbitol Oxidase of Streptomyces sp. h-7775 in Streptomyces lividans

The protein sequence (SEQ ID NO:1) of the sorbitol oxidase was obtained from the published amino acid sequence (*See e.g.,* Hiraga et al., Biosci. Biotechnol. Biochem., 62: 4347-353 [1998]). The signal sequence of the twin-arginine pathway of the *Streptomyces ceolicolor* SCO6772 gene (SEQ ID NO:3) was obtained from complete genome sequence of *Streptomyces coelicolor.*

The sorbitol oxidase was expressed in *Streptomyces* as a fusion protein of the signal sequence of the SCO6772 protein (SEQ ID NO:3) and sorbitol oxidase (SEQ ID NO: 1). A restriction site for *Nco*I was introduced at the 5' end of DNA for cloning purposes, which resulted addition of an amino acid glysine residues at position 2 (*See*, SEQ ID NO:4).

A restriction site for *Bam*HI was also introduced at the 3' end of DNA for cloning purposes. The codons of the fusion gene were optimized for expression in *Streptomyces lividans.* DNA was synthesis by Geneart. The DNA fragment spanning the two restriction sites (*i.e*., from *Nco*I to *Bam*HI ((SEQ ID NO:5)) was cloned into *Streptomyces* expression plasmid pKB105 (*See*, U.S. Pat. Appln. Ser. No. 11/303,650, filed December 16, 2005, incorporated by reference in its entirety) which was cut with *Bam*HI completely and *Nco*I partially.

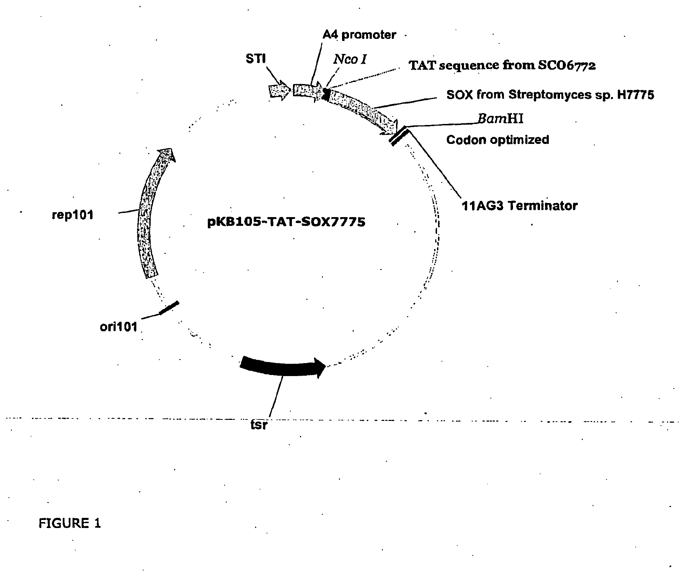

The expression plasmid (pKB105-TAT-SOX-7775; Figure 1) was transformed into *Streptomyces lividans* strain g3s3 (*See,* U.S. Pat. Appln. Ser. No. 11/305,650, *supra*) and three transformants were selected and grown in TS medium for 2- 3 days in the presence of 50 ug/ml thiostrepton at 30°C. Cells were then transferred to a production medium free of antimicrobials and growth was continued for another three days. Then, 1 ml of the culture was transferred to each of two culture tubes and the cells were removed by centrifuge under conditions sufficient to separate the cells from the supernatants. The supernatants obtained from these two culture tubes were tested in enzyme activity assays. Two oligos (SEQ ID NO:6 and SEQ ID NO:7) were obtained from Invitrogen.
GCGCTAGCCGGCCCCCCGGCACAGGCCATGACCCCGGCCGAGAAGAACTGGG (SEQ ID NO:6).
CAGGAAACAGCTATGAC (SEQ ID NO:7)

The primers were used in PCR to amplify sorbitol oxidase gene and to fuse the sorbitol oxidase gene to the ceIA signal sequence. The PCR reaction mixture containing DNA, dNTPs, primer and 4% DMSO in 1x buffer was heated to 98oC for 4 minutes to denature the DNA templates. Herculase® II enzyme (Stratagene) was added to the tube and PCR reaction was performed In 30 cycles of 98oC for 30 seconds, 62oC for 30 seconds and 72oC for 1 minute and 8 seconds. The final extension at 72°C was done for 5 minutes and the reaction was chilled to 4°C

The resulting PCR fragment contained a portion of the *ceIA* signal sequence, the sorbitol oxidase gene, and a portion of vector sequence containing two restriction enzyme sites (SEQ ID NO:8).

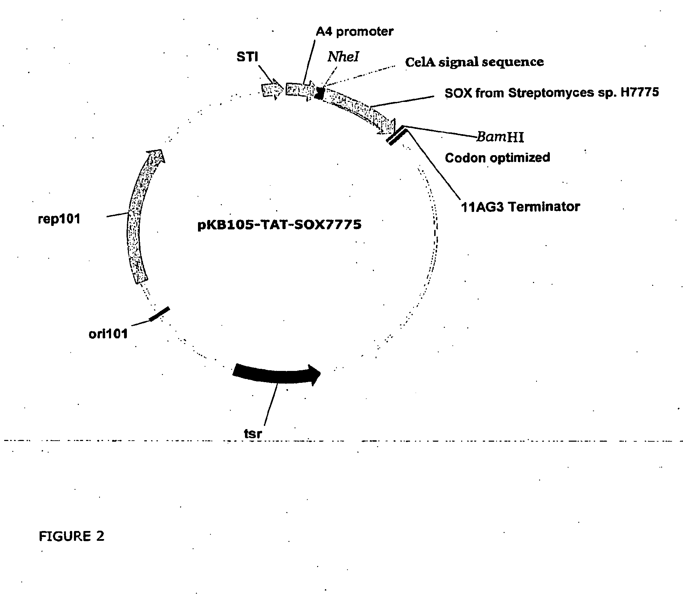

The PCR fragment was digested with restriction enzymes *Nhe*I and *Bam*HI to remove the vector sequence portion. The resulting fragment was then cloned to expression vector pKB105 to generate the plasmid "pKB105-CeIA-Sox7775" (*See*, Figure 2). The expression plasmid was transformed into *Streptomyces lividans* strain g3s3 and three transformants were selected and grown in TS medium for 2- 3 days in the presence of 50 ug/ml thiostrepton at 30°C. Cells were then transferred to a production medium free of antibiotics and growth was continued for another three days. Then, 1 ml sample was transferred to each of two new culture tubes and cells were removed and the enzyme was purified as described in Example 2.

**EXAMPLE 2**: Expression of the sorbitol oxidase gene from *Streptomyces* sp. H-7775 in *E.coli* strain BL21(DE3)pLysS.

The amino acid sequence of the sorbitol oxidase gene from *Streptomyces sp.* H-7775 SOX gene, published by Hiraga et al. 1998. Bioscience Biotech. Biochem. 61:1699-1704, 1998 was retrieved from the sequence database.

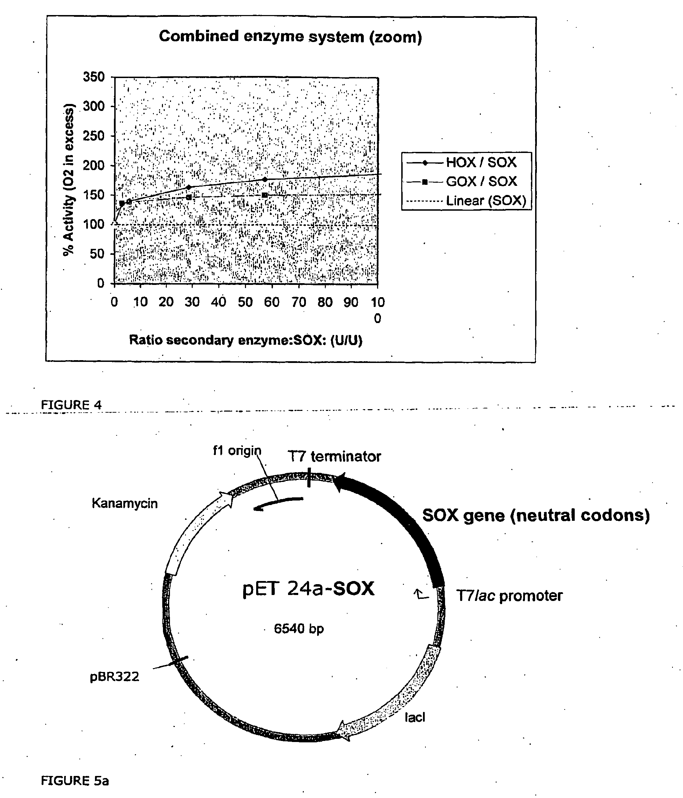

A synthetic gene (with neutral codons) encoding the H-7775 SOX gene was used to express the sorbitol oxidase gene in *E*. *coli* strain BL21(DE3)pLysS. The expression vector pET 24a with the SOX gene was cloned as Nde1 + Bam H1 fragment. The resulting plasmid (Figure. 5a.) was transformed and propagated in *E.coli* Top10 cells (Invitrogen, USA). Kanamycin resistant transformants containing the 1.2 kb SOX gene were identified by the direct colony PCR method. The SOX positive transformants were cultivated and plasmid DNA isolated. Plasmid DNA containing the cloned SOX gene was then used to transform the host strain BL21(DE3)pLysS. The entire transformation reaction was directly used to inoculate a 250ml flasks containing 25ml LB + antibiotics kanamycin (50ug/ml) and chloramphenicol (34ug/ml). The cultures were incubated overnight with shaking at 37°C. A 2 liter flasks containing 500 mls of LB containing kanamycin (50ug/ml) and chloramphenicol (34ug/ml) was inoculated with 25 mls of the overnight culture and was further allowed to grow for 2 hours to reach approximately OD₆₀₀ 0.4-0.6 mid logarithmic phase. IPTG to a final concentration of 1mM was added to the cultures. The cultures were further incubated for another 2 hours and then harvested by centrifugation. The resulting pellets were resuspended in phosphate buffer and the cells were passed through a French press for cell disruption/lysis. The different cell lysates were analyzed for sox activity (see Table 1 below) and fractionated by gel electrophoresis (native & denaturing conditions). The cell lysates were fractionated on a native gel that was further tested in an in-gel overlay activity assay using sorbitol as the substrate and an assay based on the PMS mediated reduction of NBT. Figure. 5b shows the presence of a discrete stained protein band corresponding to a flavoprotein, the SOX protein that has activity towards sorbitol. The PMS/NBT is a diagnostic assay for flavoproteins where PMS=Phenazine methosulfate is a redox mediator which is reduced by reduced FAD. Glucose oxidase is also a flavoprotein in lane 1 Fig. 5b but is not active towards sorbitol thus the absence of stained band. P10 is transformant with the empty vector pET24a (with out SOX gene) showing the absence of active SOX protein band. The absence of a stained band in lane 1 showed that glucose oxidase is not active towards sorbitol, the substrate used in the overlay assay mix. This assay can therefore be used to determine whether the first oxidase such as the polyol oxidase of the invention does not have significant activity on the second substrate.

**Table 1: SOX Activity using the (ABTS/HRP Assay) with 6 different E. coli cell extracts. P10 extract is a negative control with background activity.**

| | |
|---|---|
| 1. | Assay reagent (100 mM Kpi, pH 7, 1% Sorbitol, 5mM ABTS, 10U7ml HRP) |
| 2. | 37°C, 990µl if assay reagent |
| 3. | 3 minutes |
| 4. | P10 control = 0.2U/mg |
| 5. | P18 = 1.2U/mg |
| 6. | P6=1.05 U/mg |
| 7. | P6H = 1.49 U/mg |
| 8. | P19 = 0.69 U/mg |
| 9. | P19H = 1_{.}17 U/mg |
| 10. | *Oda* & *Hiraga E.coli* expression of SOX at cell extract stage = 1.1U/mg. |

Table 1 shows the production of sorbitol oxidase as an active enzyme in *E.coli BL21(DE3)pLysS.* The negative control showed a background activity of 0.2U/mg. The enzyme activity was improved by heating (H) the cell free extracts P6H & P19H, indicating that a heat treatment step can be used for purification of the SOX protein. The *E*. *coli* expressed SOX have specific activity similar to previous work carried out by Kohei Oda & Kazumi Hiraga (Biosci Biotech Biochem 61:1699-1704, 1997).

### EXAMPLE 3: Expression of the putative sorbitol oxidase gene from Streptomyces coelicolor/lividans in Streptomyces lividans strain S3G3

The *Streptomyces coelicolor* gl |28380233|sp|Q9ZBU1|XYOA_STRCO annotated as a probable *xylitol oxidase* (XOX) has been identified by blast searches to be the closest in sequence identity (between 54-60% depending on the alignment) to *Streptomyces sp* H-7775 sorbitol oxidase gene. The locus containing the *Streptomyces coelicolor* putative XOX gene, SCO6147, ORFNames=SC1A9.11c sequence was retrieved from the sequence database and several gene specific primers & flanking primers were used to isolate the corresponding S. *lividans* gene by PCR. The *Streptomyces lividans* complete genome sequence is not available yet but is almost identical to the fully sequenced S. coelicolor genome. Final PCR reaction consisted of Primers us-sco1 5'gcccatatgagcgacatcacggtcacc (SEQ ID NO 9) and Issco1 5' ggatcctcagcccgcgagcacccc (SEQ ID NO 10), genomic DNA from S. lividans as the template resulting in the synthesis of a 1.269 kb PCR fragment. The PCR conditions used in this final PCR step consisted of 30 cycles: denaturation at 94°C for 55 seconds, annealing at 55°C for 55 seconds, extension for 1-2 minutes at 68°C. The polymerase used is Platinum Pfx DNA polymerase plus enhancer solution from Invitrogen. The resulting PCR product was cloned directly in an E. coli vector PCR Blunt TOPO. Primers us-s1 5'gccatgggcgacatcacggtcaccaac (SEQ ID NO 11), and Is-s1 5' atggatcctcagcccgcgagcacccc (SEQ ID NO 12), were used in a PCR reaction using the same conditions as above. The -1.268kb PCR product was digested with Nco1-BamH1 and the resulting fragment was cloned directly into an Nco1+ Bam H1 digested *Streptomyces* vector pKB105. The final construct is the expression vector designated as pSMM-SOX (S. lividans) in Figure 6. The cloned SOX in PCR Blunt TOPO was used to verify the nucleotide sequence of the putative SOX gene. The 5 ul of plasmid DNA (Figure 6) was used to transform *Streptomyces* g3s3 protoplasts. Transformation reaction was plated on R5 plates and incubated at 32C for 18 hours. Soft nutrient agar overlay containing thiostrepton was poured on the plates that were further incubated for 3 days. Single colonies were used to inoculate a 250 ml flask with 20 ml TS-G media containing thiostrepton. After 3 days of cultivation with shaking at 30C, 2 ml aliquots were used to inoculate a 250 ml flask containing the Production media. The cell pellets was collected by centrifugation, resuspended in buffer and disrupted. Table 2 shows the SOX activities present in the cell-free extracts derived from the different *Streptomyces* transformants.

**Table 2. SOX activities of the cell-free extracts from 20 different Streptomyces transformants showing varying amounts of activity. Putative SOX Activity Assay (ABTS/HRP) (U/mg).**

| Transformant | 17 | 20 | 22 | 24 |
|---|---|---|---|---|
| 1 | 11.38 | 8.03 | 10.89 | 13.60 |
| 2 | 6.77 | 13.14 | 12.38 | 10.38 |
| 3 | 8.88 | 16.81 | 8.85 | 17.03 |
| 4 | 9.37 | 12.32 | 16.30 | 18.28 |
| 5 | 7.34 | 11.68 | 0.02 | 18.14 |

The DNA and protein sequences of the sorbitol oxidase from S.coelicolor (SCO6147) are shown as SEQ ID NO: 2 and 13 respectfully.

### EXAMPLE 4: Purification of Sorbitol Oxidase

In this Example, methods used to purify sorbitol oxidase produced by *S. lividans* (*See*, Example 1 - 4), are described. Sorbitol oxidase from *Streptomyces lividans* is localized in mycelia. Thus, the enzyme was isolated by cell lysis using a French press from cell-extract in 100mM Kpi buffer, potassium phosphate, pH 7.0. The cell extract was heated to 50°C for one hour, followed by centrifugation sufficient to remove the debris. The cell lysate supernatant was then mixed with ammonium sulfate to 32% saturation to precipitate the protein fraction containing sorbitol oxidase. The protein precipitate was kept at 4°C overnight and then was separated from mother liquor by centrifugation 10,000 RPM using Sorvall centrifuge and SLA-1500 Sorvall rotor.

The protein precipitate was then washed with 32% saturated ammonium sulfate solution. The washed protein precipitate was dissolved back in Kpi buffer and the insoluble material was discarded. The soluble fraction was dialyzed against 25 mM Kpi buffer, pH 7.0, overnight and then further purified using affinity chromatography on the reactive orange resin (Prometic). This partially purified sorbitol oxidase preparation and fermentation broth cell lysate (EFT of 108 hrs) were used as samples in experiments for biobleaching. The molecular weight of the enzyme was determined to be ∼45,000 Da. by SDS-PAGE gel electrophoresis. The prosthetic group is a covalently bound FAD (1mol of FAD to 1 mol of SOX).

### EXAMPLE 5: Stability and Bleaching Performance of SOX in HDL Laundry Wash Conditions

In this Example, methods to determine the stability and bleaching performance of SOX in AATCC liquid detergent laundry wash conditions are described. In these experiments, AATCC standard detergent (American Association of Textile Chemists and Colorists Heavy Duty Liquid Detergent Version 2003 without brightener; key components include linear alkane sulfonate, alcohol ethoxylate, propanediol, citric acid, fatty acid, castic soda and water; Testfabrics) is used.

Three bleachable cotton swatches with juice (STC CFT CS-15), wine (STC CFT CS-3), and tea (STC CFT BC-3) are used. The swatches are cut into 15mm circles with a textile punch (Model B equipped with a 5/8" die cutter; Model 93046; NAEF).

Single swatch disks are placed into each well of a 24-well microplate (Costar 3526). One (1) ml of washing solution pH 8, containing per liter, 1.5 ml AATCC HDL detergent, 75-100 mM sorbitol, 6gpg hardness (diluted from stock 15000 gpg hardness solution containing 1.735 M calcium chloride and 0.67 M magnesium chloride), and 0.05% TAED (tetraacetylethylenediamine, Fluka) is added to each well. Five to fifty (5-50 ul) microliters of partially purified sorbitol oxidase or sorbitol oxidase obtained from a late fermentation run (108 hr EFT "effective fermentation time") produced as described in Examples 1 and 2, is added with a positive displacement pipette to 3-8 wells in one column. Hexose oxidase or glucose oxidase is added at 0.5, 5, 50, 500, 1000 and 1500U. The control wells contained no enzyme. The microplate was covered with a plastic lid and aluminum foil and incubated at 37°C with 100rpm gentle rotation for 14 hr. The plates are then removed from the shaker and tested for the presence of hydrogen peroxide with peroxide test strips (Baker).

One hundred microliters (100 ul) of 0.1 mM sodium carbonate are added to each well to elevate the pH to 10. The microplates are incubated with rotation for another 90 min and the supernatants removed by aspiration. Each well is washed three (3) times with 1.5 ml Dulbecco's PBS, pH 7.3 and three (3) times with 1.5 ml distilled water. Each disk is removed from its well and dried overnight between sheets of paper towels and not exposed to direct light. The disks are inspected visually and then analyzed with a Reflectometer CR-200 (Minolta) calibrated on a standard white tile. The average L values are calculated as the percent soil release (% SR = 100% X (Final reflectance- Initial reflectance)/(Reflectance of a white standard-Initial reflectance).

Preliminary results confirm that the sorbitol oxidase/hexose oxidase composition is stable in a typical liquid detergent system and is able to produce effective concentration of hydrogen-peroxide in presence of its substrate sorbitol mixed with detergent and available atmospheric oxygen. In addition, the composition generated hydrogen peroxide In presence of a bleach booster (*i.e*., TAED) is able to help bleach typical colored stains such as blueberry, tea and wine.

### EXAMPLE 6: Substrate Range Study of Sorbitol Oxidase

Sorbitol oxidase obtained using the methods described in Example 3-4, was tested for finding its activity with various polyol substrates. All substrates used in the assay were 55mM in 100 mM phosphate buffer pH 7.0 at 25°C. The relative activity using sorbitol as (++++++ = 100%) is shown below in Table 1. In addition to these substrates, it is contemplated that other substrates, including, but not-limited to glycerol, will find use in the present invention.

| Compound | **Relative Activity (+, 0)** |
|---|---|
| D-Sorbitol | ++++++ |
| D-Xylitol | ++++ |
| D-Mannitol | +++ |
| D-Ribitol | + |
| Myo-Inositol | + |
| Glycerol | + |
| 1,3-propanediol | +/2 |
| 1,2-propanediol | +/2 |
| Propylene glycol | 0 |
| Ethylene glycol | 0 |

### EXAMPLE 7: Determination of SOX, HOX and GOX activity.

Glucose oxidases have the ability to oxidise glucose to yield hydrogen peroxide. Examples are carbohydrate oxidase, glucooligosaccharide oxidase and glucose oxidase. Hexose oxidase may also be classified as a glucose oxidase, although it typically has a broader specificity, with significant activity on other hexoses asn well as disaccharides, such as maltose.

### Unit definitions

1 polyol unit (POX) corresponds to the amount of enzyme, which under the specified conditions results in the conversion of 1 µmole of the specified polyol per minute, with resultant generation of 1 µmole of hydrogen peroxide (H₂O₂).

1 sorbitol oxidase (SOX) unit corresponds to the amount of enzyme, which under the specified conditions results in the conversion of 1 µmole sorbitol per minute, with resultant generation of 1 µmole of hydrogen peroxide (H₂O₂).

Definition: 1 hexose oxidase (HOX) unit corresponds to the amount of enzyme which under the specified conditions results In the conversion of 1 µmole of glucose, or alternative hexose sugar, per minute, with resultant generation of 1 µmole of hydrogen peroxide (H₂O₂).

Definition: 1 glucose oxidase (gluOX) unit corresponds to the amount of enzyme which under the specified conditions results in the conversion of 1 µmole of glucose per minute, with resultant generation of 1 µmole of hydrogen peroxide (H₂O₂).

Assay of SOX, HOX or GOX activity in microtiter plates (300 µl)

The commonly used horse radish peroxidase dye substrate ABTS was incorporated into an assay, measuring the production of H₂O₂ produced by HOX or GOX respectively. ABTS serves as a chromogenic substrate for peroxidase in combination with H₂O₂ facilitates the electron transport from the chromogenic dye, which is oxidised to an intensely green/blue compound.

### In vitro assay

An assay mixture contained 266 µl sorbitol (Sigma P-5504, 0.055 M in 0.1 M sodium phosphate buffer, pH 6.3), (or alternative substrate, e.g. xylitol), 12 µl 2,2'-Azino-bis(3-ethylbenzothiozoline-6-Sulfonic acid)(ABTS)(Sigma A-9941, 5 mg/ml aqueous solution), 12 µl peroxidase (POD)(Sigma P-6782, 0.1 mg/ml in 0.1 M sodium phosphate buffer, pH 6.3) and 10 µl enzyme (SOX or HOX) aqueous solution.

The assay is performed at 25° C.

The incubation was started by the addition of glucose. The absorbance was monitored at 405 nm in an ELISA reader. A standard curve, based on varying concentrations of H₂O₂, was used for calculation of enzyme activity according to the definition above.

The reactions as illustrated using sorbitol can be described in the following manner:

sorbitol + O₂ + → glucose + H₂O₂ (1)

glucose + O₂ + H₂O → gluconic acid + H₂O₂ (2)

H₂O₂ + 2ABTS (colorless) + 2 H⁺ → 2 H₂O+ 2ABTS (blue/green) (3)

Reaction (1) is catalysed by enzyme (SOX)
Reaction (2) is catalysed by enzyme (HOX or GOX)
Reaction (3) is catalysed by enzyme (POD)

With respect to reaction (2) GOOX and MnCO are also enzymes capable of catalysing this reaction

For '*in situ*' assays it may be necessary to dilute the matrix prior to performing the assay, so that the effect of interfering substances reacting unspecifically with the assay components become small enough to neglect. Alternatively, the matrix compostion may be prepared excluding the interfering substance(s).

### EXAMPLE 8: Relative activity on xylitol and Sorbitol

The purpose was to measure the difference in activity of sorbitol oxidase on D-sorbitol and D-xylitol. The ABTS assay was used as described previously to measure the rate of H₂O₂ production when both D-sorbitol and D-xylitol was in excess. The same amount of enzyme, as prepared in the previous examples was used in both cases. The table below shows the relative activity on the two substrates (given in percentage values). The enzyme used was as prepared in Examples 3 & 4.

| Substrate | Relative active % |
|---|---|
| D-sorbitol | 100 |
| D-xylitol | 47.6 |

### EXAMPLE 9. Synergy between the two enzymes

The Three Enzymes Used In this Example were:
1. Hexose oxidase: Hoxypure^{™} available from Danisco A/S.
2. The *S. coelicolor* (SCO6147) sorbitol oxidase was obtained as described in the previous examples 3-4.
3. Glucose oxidase: Sigma G7141

In the example below the amounts of enzyme are given as unit amounts.

A unit of HOX is the amount of enzyme that produced 1 umol H₂O₂/min when substrates are in excess.
A unit of GOX is the amount of enzyme that produced 1 umol H₂O₂/min when substrates are in excess.
A unit of SOX is the amount of enzyme that produced 1 umol H₂O₂/ min when substrates are in excess.

Note. In the examples below, only SOX will have excess substrates. The generated sugar will be the limiting factor for the secondary enzyme. As noted above the activity is given as a percentage value of SOX catalysing D-sorbitol when both D-sorbitol and oxygen is in excess.

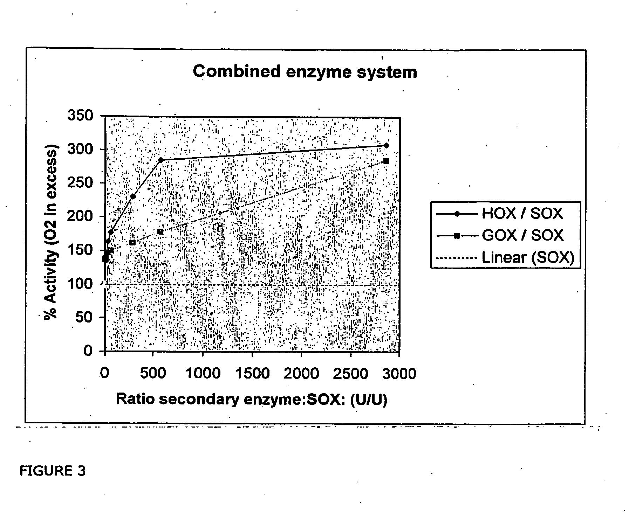

Initial velocities were measured over 5 minutes in 300 uL ABTS assay (as described previously). The production of rate of hydrogen peroxide production was extrapolated from a standard curved. The measured activity, shown in Figure 3 and Figure 4 are given as a percentage value. 100 % is defined as the rate of hydrogen peroxide production by sorbitol oxidase alone, when the substrates D-sorbitol and oxygen is in excess (Linear velocity curves)

### EXAMPLE 10: CHEWABLE TABLET

The enzyme composition is prepared by addition of 1 unit of SOX (as prepared in Examples 1-4), and either hexose oxidase or glucose oxidase is added at 0.5, 5, 50, 500, 1000 and 1500U to per microliter 100mM sodium phosphate buffer, pH 6.7, 50mM sorbitol. The aqueous enzyme coposition is used to prepare a chewable tablet. An enzyme composition containing tablet and gum compositions are prepared using conventional base ingredients as set forth below (ingredients listed in terms of wt%).

An enzyme composition containing tablet and gum compositions are prepared using conventional base ingredients as set forth below (ingredients listed in terms of wt%).

| | |
|---|---|
| Enzyme composition | 0.5% (provided as part of the water component) |
| Lycasin 75% | 48.9% |
| Isomalt or xylitol | 23.1% |
| Hydrogenated vegetable oil | 8.7% |
| Water | 4.8% |
| Gelatin (40% solution) | 2.9% |
| Starch coated dicalcium phosphate | 8. 7% |
| Mono-diglyceride mixture | 0.8% |
| Lecithin | 0.3% |
| Aspartame | 0. 05% |
| Aspartame K | 0.05% |
| Vanillin | 0.05% |
| Glycerin | 0.1% |
| Sodium bicarbonate | 0.10% |
| Mint flavor | 0.19% |

The chewable tablet Is prepared by boiling the Isomalt, Lycasin, water, fat, mono and diglyceride mixture, glycerin, and lecithin to 131°C after which glycerin is added and the mixture and cooled to 30°C (HOX) or 60°C (GOX). Thereafter sodium bicarbonate, the enzyme composition, dicalcium phosphate and the remaining ingredients are added.. Thereafter the mixture cooled to room temperature (23°C) was ground into powder and compressed into a tablet using a tablet press.

In Vivo Plaque Reduction Efficacy The chewable tablet is tested for plaque reduction at 2-and 5- hours after chewing by human volunteers using plaque grown in vivo in an inkra-oral retainer on hydroxyapatite disks. Confocal microscopy is used to visualize and quantify the changes in plaque coverage and plaque ultraskucture. Plaque removal was also measured by conventional light microscopy by staining the plaque before and after treatment with crystal violet indicator and measuring the changes in color intensity. Image Pro Analysis So aware is used to perform the image analysis and the quantitative measurements. The color intensity was measured and used to determine stain removal. The greater the intensity' the greater the cleaning efficacy.

### EXAMPLE 11: CHEWING GUM

The following ingredients may be combined to prepare a chewing gum comprising the composition of the invention:

| | |
|---|---|
| Gum base | 31.20% |
| Sorbitol | 28.08% |
| xylitol | 5.23% |
| Enzyme composition | 1.00% (see previous example) |
| Acesulfame K | 0.16% |
| Aspartame | 016% |
| Menthol powder | 1.00% |
| Liquid flavor | 0.47% |
| Isomalt PF | 11.70% |
| Isomalt DC | 16.00% |
| Anticaking agents* | 4.00% |
| Flavor | 2.00% |

| | |
|---|---|
| * Magnesium stearate, talc, silica gel. | |

### EXAMPLE 12: WATERBASED PAINT

The following ingredients may be combined to prepare a waterbased paint comprising the composition of the invention:

| | |
|---|---|
| Enzyme composition | 0.5% |
| Sorbitol | 0.5% |
| Pigment | 15% |
| Acrylic Dinder | 25% |
| Water | 50% |
| Other additives* | 9.5% |

| | |
|---|---|
| *may include the following dependiing on the type of paint: dispersion agent, suspension agent, dispersant, defoamer, wetting agent, coalescing agent, various fillers, surfactant, co-biocides, thickener, co-preservatives, latex, | |

### EXAMPLE 13: OILBASED PAINT

The following ingredients may be combined to prepare a oil based paint comprising the composition of the invention:

| | |
|---|---|
| Enzyme composition | 0.5%, dissolved in 80% sorbitol |
| TiO₂ Pigment | 20% |
| Alkyd binder | 40% |
| Hydrocarbon solvent | 20% |
| other additives* | 19.5% |

| | |
|---|---|
| *may include the following dependiing on the type of paint: dispersion agent, suspension agent, dispersant, defoamer, wetting agent, coalescing agent, various fillers, surfactant, co-biocides, thickener, co-preservatives, latex, | |

## Claims

1. A composition comprising a first oxidase, a first substrate, and at least one further oxidoreductase, wherein, the first substrate is oxidisable by the first oxidase to form hydrogen peroxide and a second substrate; and the second substrate is convertible by the at least one further oxidoreductase to form a product; wherein the second substrate is oxidisable by the further oxidoreductase to form hydrogen peroxide and said product.

2. The composition according to claim 1, wherein the first substrate is a polyol.

3. The composition according to claim 2 or 3, wherein the polyol is a pentitol or a hexitol.

4. The composition according to claim 2 or 3, wherein the polyol is selected from the group consisiting of hexitol, pentitol, sorbitol, xylitol, maltitol, mannitol, galactitol, isomalt, lactitol, arabitol, erythritol, and ribitol.

5. The composition according to claim 4, wherein the polyol is xylitol.

6. The composition according to claim 4, wherein the polyol is sorbitol.

7. The composition according to any one of claims 1 to 6 wherein the second substrate is a sugar.

8. The composition according to claim 7, wherein the sugar is a monosaccharide or a disaccharide.

9. The composition according to claim 8, wherein the sugar is selected form the group consisting of glucose, xylose, maltose, mannose, galactose, isomaltose, lactose, arabinose, erythrose and ribose.

10. The composition according to claim 9, wherein the sugar is xylose or glucose.

11. The composition according to any one of claims 1 to 10, wherein the first oxidase is a polyol oxidase.

12. The composition according to claim 11, wherein the first oxidase is selected from the group consisting of hexitol oxidase, pentitol oxidase, sorbitol oxidase, xylitol oxidase, maltitol oxidase, mannitol oxidase, galactitol oxidase, isomalt oxidase, lactitol oxidase, arabitol oxidase, arabitol oxidase, erythritol oxidase, and ribitol oxidase.

13. The composition according to claims 11 or 12, wherein the first oxidase has a polyol oxidase (specific) activity of at least 5 units/g protein when using the respective polyol substrate.

14. The composition according to any one of claims 11 to 13, wherein the polyol oxidase is sorbitol oxidase or xylitol oxidase.

15. The composition according to any one of claims 11 to 14, wherein the polyol oxidase has a ratio of specific activity on the respective polyol to the corresponding sugar of greater than 1.

16. The composition according to any one of claims 11 to 15, wherein the polyol oxidase has a ratio of specific activity on the respective polyol to glucose of greater than 1.

17. The composition according to any one of claims 11 to 16, wherein the polyol oxidase has a higher specific activity on sorbitol as compared to xylitol.

18. The composition according to any one of claims 11 to 17, wherein the polyol oxidase is derived from a strain of *Streptomyces or Xanthomonas.*

19. The composition according to any one claims 1 to 18, wherein the first oxidase comprises a polypeptide sequence SEQ ID NO 1 or SEQ ID NO 2 or a homologue, variant or fragment thereof.

20. The composition according to claim 19, wherein the first oxidase consists of a polypeptide sequence SEQ ID NO 1 or SEQ ID NO 19.

21. The composition according to any one of claims 11 to 20, wherein the level of first oxidase activity present in the composition (or added to the composition) is between about 0.1 and about 200,000 units per kg of said composition.

22. The composition according to claim any one of claims 1 - 21, wherein the further oxidoreductase is a sugar oxidase.

23. The composition according to claim 22, wherein the sugar-oxidase is selected from the group consisting of: carbohydrate oxidase, oligosaccharide oxidase, maltose oxidase, hexose oxidase, glucose oxidase, mannose oxidase, galactose oxidase, isolmaltulose oxidase, lactose oxidse, arabinose oxidase, erythrose oxidase, pentose oxidase, xylose oxidase, and triose oxidase.

24. The composition according to any one of claims 22 or 23 wherein the sugar-oxidase is selected form the group consisting of: EC 1.1.3.4 glucose oxidase, EC 1.1.3.5 hexose oxidase, EC 1.1.3.9 galactose oxidase, EC 1.1.3.10 pyranose oxidase, EC 1.1.3.11 L-sorbose oxidase, and EC 1.1.3.40 D-mannitol oxidase.

25. The composition according to any one claims 1 to 24, wherein the amount of the at least one further oxidoreductase compared to the amount of the first oxidase such as the polyol oxidase present in said composition is greater than 1, as measured by the respective number of enzyme units present in said composition.

26. The composition according to any one of claims 1 to 25, wherein the presence of the product produced from the second substrate does not reduce the rate of hydrogen peroxide production from the oxidation of the first substrate by the first oxidase.

27. The composition according to any one of claims 1 to 26, wherein the product produced from the second substrate is a lactone, a dialdose or a dehydro sugar.

28. The composition according to any one of claims 1 to 27, wherein the composition is edible, preferably a food composition.

29. A composition according to any of claims 1 to 28 for use as a medicament.

30. An oral care product comprising a composition according to any one of claims 1-29 and at least one further ingredient used in oral care products

31. An oral care product according to claim 30, wherein the oral care product is in the form selected from the group consisting of: chewing gum, a mouthwash, a mouthspray, a pastille, a lozenge, a mouth freshener, a nasal spray, and an oral paste.

32. An oral care product according to claims 30 or 31, which comprises xylitol and/or sorbitol.

33. A cosmetic product comprising the composition according to any one of claims 1-28 and at least one further ingredient used in cosmetic products

34. A skin, hair or teeth bleaching product comprising the composition according to any one of claims 1-28 and at least one further ingredient used in skin, hair or teeth bleaching and/ or whitening products.

35. A cosmetic method for bleaching and/or whitening of external mammalian tissue, comprising contacting the external mammalian tissue with a composition according to any one of claims 1-28, or a product according to any one of claims 30 to 34 in an amount and duration suitable for bleaching and/or whitening of the external mammalian tissue.

36. A cosmetic method according to claim 35, wherein the external mammalian tissue is selected from the group consisting of teeth, hair and skin.

37. A detergent or bleaching product, suitable for use on non-living tissue, comprising the composition according to any one of the claims 1-28, and at least one further ingredient used in detergent or bleaching products.

38. A paint product, such as a maritime, decorative or protective paint, which comprises the composition according to any one of claims 1 - 28, and at least one further ingredient used in paint.

39. A pesticide which comprises the composition according to any one of claims 1-28, and at least one further ingredient used in pesticides.

40. A food or feed product comprising the composition according to any one of claims 1 - 28.

41. The non-therapeutic use of a composition according any one of claims 1-28, or an oral care product according to any one of claims 30-32, for the production of hydrogen peroxide.

42. The use according to claim 41, wherein said use occurs in an oral care product to provide beneficial teeth bleaching and/or whitening effects, and/or extended shelf life, and/or anti-microbial/anti-bacterial effects either prior to or during use.

43. The use according to claim 41, wherein said use occurs in an edible product to provide beneficial prebiotic effects when consumed by an individual mammal, and/or a prolonged shelf life.

44. The use according to claim 41, wherein said use occurs in a cosmetic product to provide a prolonged shelf life, and/or is capable of bleaching and/or whitening external mammalian tissue, and/or has an anti-microbial/anti-bacterial effect when applied to the human skin.

45. The use according to claim 41, wherein said use occurs in a detergent to enhance the bleaching, whitening or disinfecting capabilities of the detergent when used on a non-living material

46. The use according to claim 41, wherein said use occurs in a paint product which shows improved preservation either before or after application.

47. The use according to claim 41, wherein said use occurs in a pesticide product which shows improved ability to prevent, reduce or kill microbial pests.

48. The use according to claim 41, wherein said use occurs in a food product, such as dairy products, such as milk, cream, cheese, whey, yoghurt, butter, or egg, such as egg yolk or egg white.

49. The use according to claim 41, wherein the composition is used for general disinfecting of food or feed, or food and feed environments, such as washing fruit and vegetables or washing and disinfecting animal carcasses and food products derived therefrom.

50. A method for the preparation of a composition according to any one of the preceding claims, comprising admixing a first oxidase as defined in any one of the preceding claims, and a first substrate according to any one of the preceding claims, and at least one further oxidoreductase according to any one of the preceding claims, and a suitable matrix.

51. A method according to claim 50, wherein the matrix is edible.

52. A method according to claim 50 or 51, wherein the composition is selected from the group consisting of a medicament, an oral care product, and a beverage.

53. A method according to claim 50 or 51, wherein the composition is a food product, such as dairy products, such as milk, cream, cheese, whey, yoghurt, butter, or egg, such as egg yolk or egg white.

54. A method according to claim 50, wherein the matrix is a detergent or bleaching ingredient or product.

55. A method according to claim 50, wherein the matrix is a paint, such as a maritime or decorative paint.

56. A method according to claim 50, wherein the matrix is a pesticide matrix

57. The use of a composition as defined in claims 1-28, in the manufacture of a medicament for the treatment or prevention of a medical disorder selected from: gum disease, gingivitis, irritable bowel syndrome, lactose intolerance, colon cancer, high blood cholesterol, high blood pressure, hypertension, infection, inflammation and nutritional deficiencies.

58. A packaged product comprising the composition according to any one of claims 1 to 28, wherein said composition is maintained in a oxygen limited environment within said packaged product so as to prevent or reduce the generation of hydrogen peroxide within said packaged product.

59. A kit of parts comprising the following components: first oxidase, at least one further oxidoreductase and a first substrate, wherein the first oxidase and the first substrate are isolated from one another, wherein, the first substrate is oxidisable by the first oxidase to form hydrogen peroxide and a second substrate; and the second substrate is convertible by the at least one further oxidoreductase to form a product; wherein the second substrate is oxidisable by the further oxidoreductase to form hydrogen peroxide and said product.

60. A non-therapeutic method of generating hydrogen peroxide, the method comprising admixing a first oxidase, and a first substrate, and at least one further oxidoreductase and a suitable matrix, under conditions suitable for the generation of both hydrogen peroxide and a second substrate from the oxidation of the first substrate due to the activity of the first oxidase, wherein the second substrate is convertable by the at least one further oxidoreductase into a product, which converts the second substrate into further hydrogen peroxide and the product.

61. The method according to claims 60, wherein the admixing step comprises mixing the components of the kit of parts according to claim 59.

62. The method according to claims 60 or 61, wherein said method comprises exposing the composition contained within the packaged product according to claim 57 to an exogenous supply of oxygen.

## Patentansprüche

1. Zusammensetzung, welche eine erste Oxidase, ein erstes Substrat, und mindestens eine weitere Oxidoreduktase umfasst, wobei das erste Substrat durch die erste Oxidase oxidierbar ist Wasserstoffperoxid und ein zweites Substrat zu bilden; und das zweite Substrat ist durch die mindestens eine weitere Oxidoreduktase umsetzbar ein Produkt zu bilden; wobei das zweite Substrat durch die weitere Oxidoreduktase oxidierbar ist Wasserstoffperoxid und das Produkt zu bilden.

2. Zusammensetzung nach Anspruch 1, wobei das erste Substrat ein Polyol ist.

3. Zusammensetzung nach Anspruch 2 oder 3, wobei das Polyol ein Pentitol oder ein Hexitol ist.

4. Zusammensetzung nach Anspruch 2 oder 3, wobei das Polyol ausgewählt ist aus der Gruppe bestehend aus Hexitol, Pentitol, Sorbitol, Xylitol, Maltitol, Mannitol, Galactitol, Isomalt, Lactitol, Arabitol, Erythritol, und Ribitol.

5. Zusammensetzung nach Anspruch 4, wobei das Polyol Xylitol ist.

6. Zusammensetzung nach Anspruch 4, wobei das Polyol Sorbitol ist.

7. Zusammensetzung nach irgendeinem der Ansprüche 1 bis 6, wobei das zweite Substrat ein Zucker ist.

8. Zusammensetzung nach Anspruch 7, wobei der Zucker ein Monosaccharid oder ein Disaccharid ist.

9. Zusammensetzung nach Anspruch 8, wobei der Zucker ausgewählt ist aus der Gruppe bestehend aus Glucose, Xylose, Maltose, Mannose, Glactose, Isomaltose, Lactose, Arabinose, Erythrose und Ribose.

10. Zusammensetzung nach Anspruch 9, wobei der Zucker Xylose oder Glucose ist.

11. Zusammensetzung nach irgendeinem der Ansprüche 1 bis 10, wobei die erste Oxidase eine Polyoloxidase ist.

12. Zusammensetzung nach Anspruch 11, wobei die erste Oxidase ausgewählt ist aus der Gruppe bestehend aus Hexitoloxidase, Pentitoloxidase, Sorbitoloxidase, Xylitoloxidase, Maltitoloxidase, Mannitoloxidase, Galactitoloxidase, Isomaltoxidase, Lactitoloxidase, Arabitoloxidase, Erythritoloxidase, und Ribitoloxidase.

13. Zusammensetzung nach Anspruch 11 oder 12, wobei die erste Oxidase eine Polyoloxidase-(spezifische) Aktivität von mindestens 5 Einheiten / g Protein aufweist, wenn das entsprechende Polyol-Substrat verwendet wird.

14. Zusammensetzung nach irgendeinem der Ansprüche 11 bis 13, wobei die Polyoloxidase Sorbitoloxidase oder Xylitoloxidase ist.

15. Zusammensetzung nach irgendeinem der Ansprüche 11 bis 14, wobei die Polyoloxidase ein Verhältnis von spezifischer Aktivität auf das entsprechende Polyol zum entsprechenden Zucker von mehr als 1 aufweist.

16. Zusammensetzung nach irgendeinem der Ansprüche 11 bis 15, wobei die Polyoloxidase ein Verhältnis von spezifischer Aktivität auf das entsprechende Polyol zu Glucose von mehr als 1 aufweist.

17. Zusammensetzung nach irgendeinem der Ansprüche 11 bis 16, wobei die Polyoloxidase eine höhere spezifische Aktivität auf Sorbitol im Vergleich zu Xylitol aufweist.

18. Zusammensetzung nach irgendeinem der Ansprüche 11 bis 17, wobei die Polyoloxidase aus einem Stamm von *Streptomyces* oder *Xanthomonas* abgeleitet ist.

19. Zusammensetzung nach irgendeinem der Ansprüche 1 bis 18, wobei die erste Oxidase eine Polypeptidsequenz SEQ ID Nr. 1 oder SEQ ID Nr. 2 oder ein Homologon, Variante oder Fragment davon umfasst.

20. Zusammensetzung nach Anspruche 19, wobei die erste Oxidase aus einer Polypeptidsequenz SEQ ID Nr. 1 oder SEQ ID Nr. 19 besteht.

21. Zusammensetzung nach irgendeinem der Ansprüche 1 bis 20, wobei die Höhe der Aktivität der ersten Oxidase in der Zusammensetzung (oder zugegeben zu der Zusammensetzung) zwischen ungefähr 0,1 und ungefähr 200.000 Einheiten pro kg der Zusammensetzung beträgt.

22. Zusammensetzung nach irgendeinem der Ansprüche 1 bis 21, wobei die weitere Oxidoreduktase eine Zuckeroxidase ist.

23. Zusammensetzung nach Anspruch 22, wobei die Zuckeroxidase ausgewählt wird aus der Gruppe bestehend aus: Kohlenhydratoxidase, Oligosaccharidoxidase, Maltoseoxidase, Hexoseoxidase, Glucoseoxidase, Mannoseoxidase, Galactoseoxidase, Isomaltuloseoxidase, Lactoseoxidase, Arabinoseoxidase, Erythroseoxidase, Pentoseoxidase, Xyloseoxidase, und Trioseoxidase.

24. Zusammensetzung nach irgendeinem der Ansprüche 22 oder 23, wobei die Zuckeroxidase ausgewählt wird aus der Gruppe bestehend aus: EC 1.1.3.4 Glucoseoxidase, EC 1.1.3.5 Hexoseoxidase, EC 1.1.3.9 Galactoseoxidase, EC 1.1.3.10 Pyranoseoxidase, EC 1.1.3.11 L-Sorboseoxidase, und EC 1.1.3.40 D-Mannitoloxidase.

25. Zusammensetzung nach irgendeinem der Ansprüche 1 bis 24, wobei die Menge von der mindestens einen weiteren Oxidoreduktase im Vergleich zu der Menge der ersten Oxidase, wie beispielsweise die Polyoloxidase in der Zusammensetzung, größer als 1 ist, was durch die entsprechende Anzahl von Enzymeinheiten in der Zusammensetzung gemessen wird.

26. Zusammensetzung nach irgendeinem der Ansprüche 1 bis 25, wobei die Anwesenheit des vom zweiten Substrat produzierten Produkts die Rate der Wasserstoffperoxid-Produktion aus der Oxidation des ersten Substrats durch die erste Oxidase nicht reduziert.

27. Zusammensetzung nach irgendeinem der Ansprüche 1 bis 26, wobei das von dem zweiten Substrat hergestellte Produkt ein Lacton, eine Dialdose oder ein Dehydro-Zucker ist.

28. Zusammensetzung nach irgendeinem der Ansprüche 1 bis 27, wobei die Zusammensetzung essbar, vorzugsweise eine Lebensmittelzusammensetzung ist.

29. Zusammensetzung nach irgendeinem der Ansprüche 1 bis 28 zur Verwendung als ein Medikament.

30. Mundpflegeprodukt, umfassend eine Zusammensetzung nach irgendeinem der Ansprüche 1 bis 29 und mindestens ein weiterer Inhaltsstoff, welcher in Mundpflegeprodukten verwendet wird.

31. Mundpflegeprodukt nach Anspruch 30, wobei das Mundpflegeprodukt in der Form vorliegt, ausgewählt aus der Gruppe bestehend aus: Kaugummi, ein Mundwasser, ein Mundspray, eine Pastillle, eine Lutschtablette, ein Munderfrischer, ein Nasenspray, und eine orale Paste.

32. Mundpflegeprodukt nach Anspruch 30 oder 31, welches Xylitol und / oder Sorbitol umfasst.

33. Kosmetisches Produkt, umfassend die Zusammensetzung nach irgendeinem der Ansprüche 1 - 28 und mindestens einem weiteren Inhaltsstoff, der in kosmetischen Produkten verwendet wird.

34. Haut-, Haar- oder Zahnbleichprodukt, umfassend die Zusammensetzung nach irgendeinem der Ansprüche 1 - 28 und mindestens ein weiterer Inhaltsstoff, der in Haut-, Haar- oder Zahnbleichprodukten und / oder Aufhellungsprodukten verwendet wird.

35. Kosmetisches Verfahren zum Bleichen und / oder Aufhellen von äußerem Säugergewebe, umfassend Inkontaktbringen von dem äußeren Säugergewebe mit einer Zusammensetzung nach irgendeinem der Ansprüche 1 - 28, oder mit einem Produkt nach irgendeinem der Ansprüche 30 bis 34 in einer Menge und Dauer, welche geeignet ist zum Bleichen und / oder Aufhellen von äußerem Säugergewebe.

36. Kosmetisches Verfahren nach Anspruch 35, wobei das äußere Säugergewebe ausgewählt wird aus der Gruppe bestehend aus Zähne, Haare und Haut.

37. Reinigungsmittel oder Bleichprodukt, was geeignet ist für nicht-lebendes Gewebe, umfassend die Zusammensetzung nach irgendeinem der Ansprüche 1 bis 28, und mindestens einem weiteren Inhaltsstoff, der in Reinigungsmitteln oder Bleichprodukten verwendet wird.

38. Lackprodukt, wie beispielsweise ein maritimer, dekorativer oder schützender Lack, welcher die Zusammensetzung nach irgendeinem der Ansprüche 1 - 28 umfasst, und mindestens ein weiterer in Lack verwendeter Inhaltsstoff.

39. Pestizid, welches die Zusammensetzung nach irgendeinem der Ansprüche 1 -28 umfasst., und mindestens ein weiterer in Pestiziden verwendeter Inhaltsstoff.

40. Lebens- oder Futtermittel, welches die Zusammensetzung nach irgendeinem der Ansprüche 1 - 28 umfasst.

41. Nicht-therapeutische Verwendung einer Zusammensetzung nach irgendeinem der Ansprüche 1 - 28, oder ein Mundpflegeprodukt nach irgendeinem der Ansprüche 30 - 32, zur Herstellung von Wasserstoffperoxid.

42. Verwendung nach Anspruch 41, wobei die Verwendung in einem Mundpflegeprodukt erfolgt, um ein vorteilhaftes Zahnbleichen und / oder Aufhellungseffekte, und / oder verlängerte Haltbarkeit, und / oder eine antimikrobielle / antibakterielle Wirkung entweder vor oder während der Verwendung bereitzustellen.

43. Verwendung nach Anspruch 41, wobei die Verwendung in einem essbaren Produkt erfolgt, um vorteilhafte prebiotische Wirkungen bereitzustellen, wenn es von einem Säugerindividuum konsumiert wird, und / oder um eine verlängerte Haltbarkeit bereitzustellen.

44. Verwendung nach Anspruch 41, wobei die Verwendung in einem kosmetischen Produkt erfolgt, um eine verlängerte Haltbarkeit bereitzustellen, und / oder ist zum Bleichen oder Aufhellen von äußerem Säugergewebe in der Lage, und / oder hat eine antimikrobielle / antibakterielle Wirkung, wenn es auf die humane Haut aufgebracht wird.

45. Verwendung nach Anspruch 41, wobei die Verwendung in einem Reinigungsmittel erfolgt, um die Fähigkeiten des Reinigungsmittels zum Bleichen, Aufhellen oder Desinfizieren zu verstärken, wenn es auf nicht-lebendem Material verwendet wird.

46. Verwendung nach Anspruch 41, wobei die Verwendung in einem Lackprodukt erfolgt, welches eine verbesserte Konservierung entweder vor oder nach der Anwendung zeigt.

47. Verwendung nach Anspruch 41, wobei die Verwendung in einem Pestizidprodukt erfolgt, welches eine verbesserte Fähigkeit zeigt mikrobielle Schädlinge zu verhindern, zu reduzieren oder zu töten.

48. Verwendung nach Anspruch 41, wobei die Verwendung in einem Lebensmittel erfolgt, wie beispielsweise Molkereiprodukte, wie beispielsweise Milch, Sahne, Käse, Molke, Joghurt, Butter, oder Ei, wie beispielsweise Eidotter oder Eiweiß.

49. Verwendung nach Anspruch 41, wobei die Zusammensetzung zur üblichen Desinfektion von Lebensmitteln oder Futter, oder Lebensmittel- und Futterumfeld, wie beispielsweise Waschen von Früchten und Gemüse oder Waschen und Desinfizieren von Tierkadaver und daraus hergestellte Lebensmittel, verwendet wird.

50. Verfahren zur Herstellung einer Zusammensetzung nach irgendeinem der vorangehenden Ansprüche, welches das Beimischen einer ersten Oxidase gemäß irgendeinem der vorangehenden Ansprüche, und einem ersten Substrat nach irgendeinem der vorangehenden Ansprüche, und mindestens einer weiteren Oxidoreduktase nach irgendeinem der vorangehenden Ansprüche, und eine geeignete Matrix umfasst.

51. Verfahren nach Anspruch 50, wobei die Matrix essbar ist.

52. Verfahren nach Anspruch 50 oder 51, wobei die Zusammensetzung ausgewählt wird aus der Gruppe bestehend aus einem Medikament, einem Mundpflegeprodukt, und einem Getränk.

53. Verfahren nach Anspruch 50 oder 51, wobei die Zusammensetzung ein Lebensmittelprodukt ist, wie beispielsweise Molkereiprodukte, wie beispielsweise Milch, Sahne, Käse, Molke, Joghurt, Butter, oder Ei, wie beispielsweise Eidotter oder Eiweiß.

54. Verfahren nach Anspruch 50, wobei die Matrix ein Reinigungsmittel- oder Aufhellungsinhaltsstoff oder -produkt ist.

55. Verfahren nach Anspruch 50, wobei die Matrix ein Lack, beispielsweise ein maritimer oder dekorativer Lack ist.

56. Verfahren nach Anspruch 50, wobei die Matrix eine Pestizid-Matrix ist.

57. Verwendung einer Zusammensetzung gemäß den Ansprüchen 1 - 28, für die Herstellung eines Medikaments zur Behandlung oder Prävention einer medizinischen Störung ausgewählt aus: Zahnfleischerkrankung, Zahnfleischentzündung, Reizkolon, Laktoseintoleranz, Darmkrebs, erhöhtes Blutcholesterin, Bluthochdruck, Hypertonie, Infektion, Entzündung und Ernährungsmängel.

58. Verpacktes Produkt, welches die Zusammensetzung nach irgendeinem der Ansprüche 1 bis 28 umfasst, wobei die Zusammensetzung innerhalb des verpackten Produkts in einer Umgebung mit begrenztem Sauerstoffgehalt gehalten wird, um die Bildung von Wasserstoffperoxid innerhalb des verpackten Produkts zu verhindern oder zu reduzieren.

59. Kit mit Teilen, welche die folgenden Bestandteilen umfasst: erste Oxidase, mindestens eine weitere Oxidoreduktase und ein erstes Substrat, wobei die erste Oxidase und das erste Substrat voneinander getrennt sind, wobei das erste Substrat durch die erste Oxidase oxidierbar ist Wasserstoffperoxid und ein zweites Substrat zu bilden; und das zweite Substrate ist durch die mindestens eine weitere Oxidoreduktase umsetzbar ein Produkt zu bilden; wobei das zweite Substrat durch die weitere Oxidoreduktase oxidierbar ist Wasserstoffperoxid und das Produkt zu bilden.

60. Nicht-therapeutisches Verfahren zur Bildung von Wasserstoffperoxid, wobei das Verfahren das Beimischen einer ersten Oxidase, und eines ersten Substrats, und mindestens eine weitere Oxidoreduktase und eine geeignete Matrix umfasst, unter Bedingungen, die geeignet sind zur Bildung von sowohl Wasserstoffperoxid als auch einem zweiten Substrat aus der Oxidation des ersten Substrats aufgrund der Aktivität der ersten Oxidase, wobei das zweite Substrat durch die mindestens eine weitere Oxidoreduktase umsetzbar ist in ein Produkt, welches das zweite Substrat in weiteres Wasserstoffperoxid und das Produkt umwandelt.

61. Verfahren nach Anspruch 60, wobei der Beimischungsschritt das Mischen der Bestandteile des Kits mit Teilen nach Anspruch 59 umfasst.

62. Verfahren nach Anspruch 60 oder 61, wobei das Verfahren umfasst, dass die Zusammensetzung, die innerhalb des verpackten Produkts nach Anspruch 57 enthalten ist, von außen zugeführtem Sauerstoff ausgesetzt wird.

## Revendications

1. Une composition comprenant une première oxydase, un premier substrat et au moins une autre oxydoréductase, dans laquelle le premier substrat est oxydable par la première oxydase pour former du peroxyde d'hydrogène et un second substrat ; et le second substrat est convertible par l'autre oxydoréductase pour former un produit ; dans lequel ledit second substrat est oxydable par l'autre oxydoréductase pour former le peroxyde d'hydrogène et ledit produit.

2. La composition selon la revendication 1, dans laquelle le premier substrat est un polyol.

3. La composition selon la revendication 2 ou 3, dans laquelle le polyol est un pentitol ou un hexitol.

4. La composition selon la revendication 2 ou 3, dans laquelle le polyol est choisi dans le groupe constitué par l'hexitol, le pentitol, le sorbitol, le xylitol, le maltitol, le mannitol, le galactitol, l'isomalt, le lactitol, l'arabitl, l'erythritol et le ribitol.

5. La composition selon la revendication 4, dans laquelle le polyol est le xylitol.

6. La composition selon la revendication 4, dans laquelle le polyol est le sorbitol.

7. La composition selon l'une des revendications 1 à 6, dans laquelle le second substrat est un sucre.

8. La composition selon la revendication 7, dans laquelle le sucre est un monosaccharide ou un disaccharide.

9. La composition selon la revendication 8, dans laquelle le sucre est choisi dans le groupe constitué par le glucose, le xylose, le maltose, le mannose, le galactose, l'isomaltose, le lactose, l'arabinose l'érythrose et le ribose.

10. La composition selon la revendication 9, dans laquelle le sucre est le xylose ou le glucose.

11. la composition selon l'une quelconque des revendications 1 à 10, dans laquelle la première oxydase est une polyol-oxydase.

12. La composition selon la revendication 11, dans laquelle la première oxydase est choisie dans le groupe constitué par l'hexitol-oxydase, la pentitol-oxydase, la sorbitol-oxydase, la xylitol-oxydase, la maltitol-oxydase, la mannitol-oxydase, la galactitol-oxydase, l'isomalt-oxydase, la lactitol-oxydase, l'arabitol-oxydase, l'arabitol-oxydase, l'érythritol-oxydase et la ribitol-oxydase.

13. La composition selon les revendications 11 ou 12, dans laquelle la première oxydase est une polyol-oxydase à activité (spécifique) d'au moins 5 unités/g de protéine lorsqu'on utilise le substrat polyol respectif.

14. La composition selon l'une quelconque des revendications 11 à 13, dans laquelle la polyol-oxydase est la sorbitol-oxydase ou la xylitol-oxydase.

15. La composition selon l'une quelconque des revendications 11 à 14, dans laquelle la polyol-oxydase a un rapport, de l'activité spécifique vis-à-vis du polyol respectif sur le sucre correspondant, supérieur à 1.

16. La composition selon l'une quelconque des revendications 11 à 15, dans laquelle la polyol-oxydase a un rapport, de l'activité spécifique vis-à-vis du polyol respectif sur le glucose, supérieur à 1.

17. La composition selon l'une quelconque des revendications 11 à 16, dans laquelle la polyol-oxydase a une activité spécifique sur le sorbitol supérieure à celle sur le xylitol.

18. La composition selon l'une quelconque des revendications 11 à 17, dans laquelle la polyol-oxydase est dérivée d'une couche de *Streptomyces* ou *Xanthomonas.*

19. La composition selon l'une quelconque des revendications 1 à 18, dans laquelle la première oxydase comprend une séquence de polypeptide SEQ ID NO 1 ou SEQ ID NO 2 ou un homologue, une variante ou un fragment de celle-ci.

20. La composition selon la revendication 19, dans laquelle la première oxydase est constituée d'une séquence de polypeptide SEQ ID NO 1 ou SEQ ID NO 19.

21. La composition selon l'une quelconque des revendications 11 ou 20, dans laquelle le niveau d'activité de la première oxydase présente dans la composition (ou ajoutée à la composition) est compris entre environ 0,1 et environ 200 000 unités par kg de ladite composition.

22. La composition selon l'une quelconque des revendications 1 à 21, dans laquelle l'autre oxydoréductase est une oxydase de sucre.

23. La composition selon la revendication 22, dans laquelle l'oxydase de sucre est choisie dans le groupe constitué par l'oxydase de carbohydrate, l'oxydase d'oligosaccharide, l'oxydase de maltose, l'oxydase d'hexose, l'oxydase de glucose, l'oxydase de mannose, l'oxydase de galactose, l'oxydase d'isolmaltulose, l'oxydase de lactose, l'oxydase d'arabinose, l'oxydase d'érythrose, l'oxydase de pentose, l'oxydase de xylose et l'oxydase de triose.

24. La composition selon l'une quelconque des revendications 22 et 23, dans laquelle l'oxydase de sucre est choisie dans le groupe constitué par l'oxydase de glucose EC 1.1.3.4, l'oxydase d'hexose EC 1.1.3.5, l'oxydase de galactose EC 1.1.3.9, l'oxydase de pyranose EC 1.1.3.10, l'oxydase de L-sorbose EC 1.1.3.11 et l'oxydase de D-mannitol EC 1.1.3.40.

25. La composition selon l'une quelconque des revendications 1 à 24, dans laquelle la quantité de l'autre oxydase, comparée à la quantité de la première oxydase telle que l'oxydase-polyol présente dans la composition, est supérieure à 1, telle que mesurée par le nombre respectif d'unités d'enzyme présentes dans ladite composition.

26. La composition selon l'une quelconque des revendications 1 à 25, dans laquelle la présence du produit produit par le second substrat ne réduit pas le taux de production de peroxyde d'hydrogène provenant de l'oxydation du premier substrat par la première oxydase.

27. La composition selon l'une quelconque des revendications 1 à 26, dans laquelle le produit produit à partir du second substrat est une lactone, un dialdose ou un sucre déshydrogéné.

28. La composition selon l'une quelconque des revendications 1 à 27, dans laquelle la composition est comestible, de préférence en tant que composition alimentaire.

29. Une composition selon l'une quelconque des revendications 1 à 28, pour une utilisation comme médicament.

30. Un produit de soin buccal, comprenant une composition selon l'une quelconque des revendications 1 à 29 et au moins un autre ingrédient utilisé dans les produits de soin buccal.

31. Un produit de soin buccal selon la revendication 30, dans lequel le produit de soin buccal est sous une forme choisie dans le groupe constitué par une gomme à mâcher, un bain de bouche, une pulvérisation buccale, une tablette, une pastille, un rince-bouche, un spray nasal et une pâte buccale.

32. Un produit de soin buccal selon la revendication 30 ou 31, comprenant du xylitol et/ou du sorbitol.

33. Un produit cosmétique comprenant la composition selon l'une quelconque des revendications 1 à 28 et au moins un autre ingrédient utilisé dans les produits cosmétiques.

34. Un produit de décoloration pour la peau, les cheveux ou les dents comprenant la composition selon l'une quelconque des revendications 1 à 28 et au moins un autre ingrédient utilisé dans les produits de décoloration et/ou de blanchissement de la peau, des cheveux ou des dents.

35. Un procédé cosmétique pour la décoloration et/ou le blanchissement d'un tissu de mammifère externe, comprenant la mise en contact du tissu de mammifère externe avec une composition selon l'une quelconque des revendications 1 à 28, ou un produit selon l'une quelconque des revendications 30 à 34 en une quantité et pendant une durée appropriées pour la décoloration et/ou le blanchissement du tissu de mammifère externe.

36. Un procédé cosmétique selon la revendication 35, dans lequel le tissu de mammifère externe est choisi dans le groupe constitué par les dents, les cheveux et la peau.

37. Un détergent ou un produit de décoloration approprié pour une utilisation sur un tissu non vivant, comprenant la composition selon l'une quelconque des revendications 1 à 28 et au moins un autre ingrédient utilisé dans les détergents ou produits de décoloration.

38. Une peinture, telle qu'une peinture maritime, décorative ou protectrice, comprenant la composition selon l'une quelconque des revendications 1 à 28 et au moins un autre ingrédient utilisé dans les peintures.

39. Un pesticide comprenant la composition selon l'une quelconque des revendications 1 à 28 et au moins un autre ingrédient utilisé dans les pesticides.

40. Un produit alimentaire ou destiné à l'alimentation animale comprenant la composition selon l'une quelconque des revendications 1 à 28.

41. L'utilisation non thérapeutique d'une composition selon l'une quelconque des revendications 1 à 28, ou un produit de soin buccal selon l'une quelconque des revendications 30 à 32, pour la production de peroxyde d'hydrogène.

42. L'utilisation selon la revendication 41, dans laquelle ladite utilisation s'effectue dans un produit de soin buccal pour produire des effets bénéfiques de décoloration et/ou de blanchiment des dents et/ou une durée de vie allongée et/ou des effets anti-microbiens/anti-bactériens soit avant, soit pendant l'utilisation.

43. L'utilisation selon la revendication 41, dans laquelle ladite utilisation s'effectue dans un produit comestible pour produire des effets bénéfiques pré-biotiques lorsque le produit est consommé par un mammifère, et/ou une durée de vie allongée.

44. L'utilisation selon la revendication 41, dans laquelle ladite utilisation s'effectue dans un produit cosmétique pour obtenir une durée de vie allongée et/ou est capable de décolorer et/ou de blanchir un tissu de mammifère externe et/ou a un effet anti-microbien/anti-bactérien lorsqu'il est appliqué sur la peau humaine.

45. L'utilisation selon la revendication 41, dans laquelle ladite utilisation s'effectue dans un détergent pour améliorer la décoloration, le blanchiment ou les propriétés de désinfection du détergent lorsqu'il est utilisé sur un matériau non vivant.

46. L'utilisation selon la revendication 41, dans laquelle ladite utilisation s'effectue dans une peinture qui présente une conservation améliorée soit avant soit après l'application.

47. L'utilisation selon la revendication 41, dans laquelle ladite utilisation s'effectue dans un pesticide qui présente une aptitude améliorée à prévenir, réduire ou tuer les organismes microbiens.

48. L'utilisation selon la revendication 41, dans laquelle ladite utilisation s'effectue dans un produit alimentaire, tel qu'un produit laitier, tel que du lait, de la crème, du fromage, du lactosérum, du yaourt, du beurre et un oeuf, tel qu'un jaune d'oeuf ou un blanc d'oeuf.

49. L'utilisation selon la revendication 41, dans laquelle ladite composition est utilisée pour la désinfection générale d'aliments pour l'homme ou les animaux, ou d'environnement humains ou animaux, tels que le lavage des fruits et légumes ou le lavage et la désinfection de carcasses d'animaux et de produits alimentaires qui en dérivent.

50. Un procédé de préparation d'une composition selon l'une quelconque des revendications précédentes, comprenant le mélange d'une premier oxydase tel que définie dans l'une quelconque des revendications précédentes, et un premier substrat selon l'une quelconque des revendications précédentes et au moins une autre oxydoréductase selon l'une quelconque des revendications précédentes, et une matrice appropriée.

51. Un procédé selon la revendication 50, dans lequel la matrice est comestible.

52. Un procédé selon la revendication 50 ou 51, dans lequel la composition est choisie dans le groupe constitué par un médicament, un produit de soin buccal et une boisson.

53. Un procédé selon la revendication 50 ou 51, dans lequel la composition est un produit alimentaire tel qu'un produit laitier tel que du lait, de la crème, du fromage, du lactosérum, un yaourt, du beurre ou un oeuf, tel qu'un blanc d'oeuf ou un jaune d'oeuf.

54. Un procédé selon la revendication 50, dans lequel la matrice est un détergent ou un produit ou un ingrédient de décoloration.

55. Un procédé selon la revendication 50, dans lequel la matrice est une peinture telle qu'une peinture maritime ou décorative.

56. Un procédé selon la revendication 50, dans lequel la matrice est une matrice pesticide.

57. L'utilisation d'une composition telle que définie dans les revendications 1 à 28, dans la fabrication d'un médicament pour le traitement ou la prévention d'un trouble médical choisi parmi : les gingivites, le syndrome du colon irritable, l'intolérance au lactose, le cancer du colon, le taux de cholestérol sanguin élevé, la tension élevée, l'hypertension, une inflammation et les carences alimentaires.

58. Un produit emballé comprenant la composition selon l'une quelconque des revendications 1 à 28, dans lequel ladite composition est maintenue dans un environnement limité en oxygène à l'intérieur dudit produit emballé de façon à prévenir ou réduire la génération de peroxyde d'hydrogène à l'intérieur dudit produit emballé.

59. A paquet comprenant les composants suivants : une première oxydase, au moins une autre oxydoréductase et un premier substrat, dans lequel ladite première oxydase et le premier substrat sont isolés l'une de l'autre, et dans lequel le premier substrat est oxydable par la première oxydase pour former du peroxyde d'hydrogène, et un second substrat ; et le second substrat est convertible par l'autre oxydoréductase pour former un produit ; dans lequel le second substrat est oxydable par l'autre oxydoréductase pour former du peroxyde d'hydrogène et ledit produit.

60. Un procédé non thérapeutique de génération de peroxyde d'hydrogène, le procédé comprenant le mélange d'une première oxydase et d'un premier substrat, et d'au moins une autre oxydoréductase et une matrice appropriée, dans des conditions appropriées pour la génération à la fois de peroxyde d'hydrogène et d'un second substrat à partir de l'oxydation du premier substrat due à l'activité de la première oxydase, dans lequel le second substrat est convertible par l'autre oxydoréductase en un produit, lequel convertit le second substrat en davantage de peroxyde d'hydrogène et en le produit.

61. Le procédé selon la revendication 60, dans lequel l'étape de mélange comprend le mélange des composants du paquet selon la revendication 59.

62. Le procédé selon la revendication 60 ou 61, dans lequel ledit procédé comprend l'exposition de la composition contenue dans ledit produit emballé selon la revendication 57 à un apport d'oxygène exogène.


### SEQUENCE LISTING

<110> Dansico A/S
<120> A composition comprising a coupled enzyme system
<130> 16318PCT00
<160> 13
<170> PatentIn version 3.3
<210> 1
   <211> 420
   <212> PRT
   <213> Streptomyces sp. H7775
<400> 1
   Met Thr Pro Ala Glu Lys Asn Trp Ala Gly Asn Ile Thr Phe Gly Ala
   1               5                   10                  15
   Lys Arg Leu Cys Val Pro Arg Ser Val Arg Glu Leu Arg Glu Thr Val
               20                  25                  30
   Ala Ala Ser Gly Ala Val Arg Pro Leu Gly Thr Arg His Ser Phe Asn
           35                  40                  45
   Thr Val Ala Asp Thr Ser Gly Asp His Val Ser Leu Ala Gly Leu Pro
       50                  55                  60
   Arg Val Val Asp Ile Asp Val Pro Gly Arg Ala Val Ser Leu Ser Ala
   65                  70                  75                  80
   Gly Leu Arg Phe Gly Glu Phe Ala Ala Glu Leu His Ala Arg Gly Leu
                   85                  90                  95
   Ala Leu Ala Asn Leu Gly Ser Leu Pro His Ile Ser Val Ala Gly Ala
               100                 105                 110
   Val Ala Thr Gly Thr His Gly Ser Gly Val Gly Asn Arg Ser Leu Ala
           115                 120                 125
   Gly Ala Val Arg Ala Leu Ser Leu Val Thr Ala Asp Gly Glu Thr Arg
       130                 135                 140
   Thr Leu Arg Arg Thr Asp Glu Asp Phe Ala Gly Ala Val Val Ser Leu
   145                 150                 155                 160
   Gly Ala Leu Gly Val Val Thr Ser Leu Glu Leu Asp Leu Val Pro Ala
                   165                 170                 175
   Phe Glu Val Arg Gln Trp Val Tyr Glu Asp Leu Pro Glu Ala Thr Leu
               180                 185                 190
   Ala Ala Arg Phe Asp Glu Val Met Ser Ala Ala Tyr Ser Val Ser Val
           195                 200                 205
   Phe Thr Asp Trp Arg Pro Gly Pro Val Gly Gln Val Trp Leu Lys Gln
       210                 215                 220
   Arg Val Gly Asp Glu Gly Ala Arg Ser Val Met Pro Ala Glu Trp Leu
   225                 230                 235                 240
   Gly Ala Arg Leu Ala Asp Gly Pro Arg His Pro Val Pro Gly Met Pro
                   245                 250                 255
   Ala Gly Asn Cys Thr Ala Gln Gln Gly Val Pro Gly Pro Trp His Glu
               260                 265                 270
   Arg Leu Pro His Phe Arg Met Glu Phe Thr Pro Ser Asn Gly Asp Glu
           275                 280                 285
   Leu Gln Ser Glu Tyr Phe Val Ala Arg Ala Asp Ala Val Ala Ala Tyr
       290                 295                 300
   Glu Ala Leu Ala Arg Leu Arg Asp Arg Ile Ala Pro Val Leu Gln Val
   305                 310                 315                 320
   Ser Glu Leu Arg Thr Val Ala Ala Asp Asp Leu Trp Leu Ser Pro Ala
                   325                 330                 335
   His Gly Arg Asp Ser Val Ala Phe His Phe Thr Trp Val Pro Asp Ala
               340                 345                 350
   Ala Ala Val Ala Pro Val Ala Gly Ala Ile Glu Glu Ala Leu Ala Pro
           355                 360                 365
   Phe Gly Ala Arg Pro His Trp Gly Lys Val Phe Ser Thr Ala Pro Glu
       370                 375                 380
   Val Leu Arg Thr Leu Tyr Pro Arg Tyr Ala Asp Phe Glu Glu Leu Val
   385                 390                 395                 400
   Gly Arg His Asp Pro Glu Gly Thr Phe Arg Asn Ala Phe Leu Asp Arg
                   405                 410                 415
   Tyr Phe Arg Arg
               420
<210> 2
   <211> 418
   <212> PRT
   <213> Streptomyces coelicolor
<400> 2
   Met Gly Asp Ile Thr Val Thr Asn Trp Ala Gly Asn Ile Thr Tyr Thr
   1               5                   10                  15
   Ala Lys Glu Leu Leu Arg Pro His Ser Leu Asp Ala Leu Arg Ala Leu
               20                  25                  30
   Val Ala Asp Ser Ala Arg Val Arg Val Leu Gly Ser Gly His Ser Phe
           35                  40                  45
   Asn Glu Ile Ala Glu Pro Gly Asp Gly Gly Val Leu Leu Ser Leu Ala
       50                  55                  60
   Gly Leu Pro Ser Val Val Asp Val Asp Thr Ala Ala Arg Thr Val Arg
   65                  70                  75                  80
   Val Gly Gly Gly Val Arg Tyr Ala Glu Leu Ala Arg Val Val His Ala
                   85                  90                  95
   Arg Gly Leu Ala Leu Pro Asn Met Ala Ser Leu Pro His Ile Ser Val
               100                 105                 110
   Ala Gly Ser Val Ala Thr Gly Thr His Gly Ser Gly Val Gly Asn Gly
           115                 120                 125
   Ser Leu Ala Ser Val Val Arg Glu Val Glu Leu Val Thr Ala Asp Gly
       130                 135                 140
   Ser Thr Val Val Ile Ala Arg Gly Asp Glu Arg Phe Gly Gly Ala Val
   145                 150                 155                 160
   Thr Ser Leu Gly Ala Leu Gly Val Val Thr Ser Leu Thr Leu Asp Leu
                   165                 170                 175
   Glu Pro Ala Tyr Glu Met Glu Gln His Val Phe Thr Glu Leu Pro Leu
               180                 185                 190
   Ala Gly Leu Asp Pro Ala Thr Phe Glu Thr Val Met Ala Ala Ala Tyr
           195                 200                 205
   Ser Val Ser Leu Phe Thr Asp Trp Arg Ala Pro Gly Phe Arg Gln Val
       210                 215                 220
   Trp Leu Lys Arg Arg Thr Asp Arg Pro Leu Asp Gly Phe Pro Tyr Ala
   225                 230                 235                 240
   Ala Pro Ala Ala Glu Lys Met His Pro Val Pro Gly Met Pro Ala Val
                   245                 250                 255
   Asn Cys Thr Glu Gln Phe Gly Val Pro Gly Pro Trp His Glu Arg Leu
               260                 265                 270
   Pro His Phe Arg Ala Glu Phe Thr Pro Ser Ser Gly Ala Glu Leu Gln
           275                 280                 285
   Ser Glu Tyr Leu Met Pro Arg Glu His Ala Leu Ala Ala Leu His Ala
       290                 295                 300
   Met Asp Ala Ile Arg Glu Thr Leu Ala Pro Val Leu Gln Thr Cys Glu
   305                 310                 315                 320
   Ile Arg Thr Val Ala Ala Asp Ala Gln Trp Leu Ser Pro Ala Tyr Gly
                   325                 330                 335
   Arg Asp Thr Val Ala Ala His Phe Thr Trp Val Glu Asp Thr Ala Ala
               340                 345                 350
   Val Leu Pro Val Val Arg Arg Leu Glu Glu Ala Leu Val Pro Phe Ala
           355                 360                 365
   Ala Arg Pro His Trp Gly Lys Val Phe Thr Val Pro Ala Gly Glu Leu
       370                 375                 380
   Arg Ala Leu Tyr Pro Arg Leu Ala Asp Phe Gly Ala Leu Ala Gly Ala
   385                 390                 395                 400
   Leu Asp Pro Ala Gly Lys Phe Thr Asn Ala Phe Val Arg Gly Val Leu
                   405                 410                 415
   Ala Gly
<210> 3
   <211> 47
   <212> PRT
   <213> Streptomyces coelicolor
<400> 3
   Met Thr Glu Val Ser Arg Arg Lys Leu Met Lys Gly Ala Ala Val Ser
   1               5                   10                  15
   Gly Gly Ala Leu Ala Leu Pro Ala Leu Gly Ala Pro Pro Ala Thr Ala
               20                  25                  30
   Ala Pro Ala Ala Gly Pro Glu Asp Leu Pro Gly Pro Ala Ala Ala
           35                  40                  45
<210> 4
   <211> 468
   <212> PRT
   <213> Streptomyces sp. H7775
<400> 4
   Met Gly Thr Glu Val Ser Arg Arg Lys Leu Met Lys Gly Ala Ala Val
   1               5                   10                  15
   Ser Gly Gly Ala Leu Ala Leu Pro Ala Leu Gly Ala Pro Pro Ala Thr
               20                  25                  30
   Ala Ala Pro Ala Ala Gly Pro Glu Asp Leu Pro Gly Pro Ala Ala Ala
           35                  40                  45
   Met Thr Pro Ala Glu Lys Asn Trp Ala Gly Asn Ile Thr Phe Gly Ala
       50                  55                  60
   Lys Arg Leu Cys Val Pro Arg Ser Val Arg Glu Leu Arg Glu Thr Val
   65                  70                  75                  80
   Ala Ala Ser Gly Ala Val Arg Pro Leu Gly Thr Arg His Ser Phe Asn
                   85                  90                  95
   Thr Val Ala Asp Thr Ser Gly Asp His Val Ser Leu Ala Gly Leu Pro
   100 105 110
   Arg Val Val Asp Ile Asp Val Pro Gly Arg Ala Val Ser Leu Ser Ala
   115 120 125
   Gly Leu Arg Phe Gly Glu Phe Ala Ala Glu Leu His Ala Arg Gly Leu
   130 135 140
   Ala Leu Ala Asn Leu Gly Ser Leu Pro His Ile Ser Val Ala Gly Ala
   145 150 155 160
   Val Ala Thr Gly Thr His Gly Ser Gly Val Gly Asn Arg Ser Leu Ala
   165 170 175
   Gly Ala Val Arg Ala Leu Ser Leu Val Thr Ala Asp Gly Glu Thr Arg
   180 185 190
   Thr Leu Arg Arg Thr Asp Glu Asp Phe Ala Gly Ala Val Val Ser Leu
   195 200 205
   Gly Ala Leu Gly Val Val Thr Ser Leu Glu Leu Asp Leu Val Pro Ala
   210 215 220
   Phe Glu Val Arg Gln Trp Val Tyr Glu Asp Leu Pro Glu Ala Thr Leu
   225 230 235 240
   Ala Ala Arg Phe Asp Glu Val Met Ser Ala Ala Tyr Ser Val Ser Val
   245 250 255
   Phe Thr Asp Trp Arg Pro Gly Pro Val Gly Gln Val Trp Leu Lys Gln
   260 265 270
   Arg Val Gly Asp Glu Gly Ala Arg Ser Val Met Pro Ala Glu Trp Leu
   275 280 285
   Gly Ala Arg Leu Ala Asp Gly Pro Arg His Pro Val Pro Gly Met Pro
   290 295 300
   Ala Gly Asn Cys Thr Ala Gln Gln Gly Val Pro Gly Pro Trp His Glu
   305 310 315 320
   Arg Leu Pro His Phe Arg Met Glu Phe Thr Pro Ser Asn Gly Asp Glu
   325 330 335
   Leu Gln Ser Glu Tyr Phe Val Ala Arg Ala Asp Ala Val Ala Ala Tyr
   340 345 350
   Glu Ala Leu Ala Arg Leu Arg Asp Arg Ile Ala Pro Val Leu Gln Val
   355 360 365
   Ser Glu Leu Arg Thr Val Ala Ala Asp Asp Leu Trp Leu Ser Pro Ala
   370 375 380
   His Gly Arg Asp Ser Val Ala Phe His Phe Thr Trp Val Pro Asp Ala
   385 390 395 400
   Ala Ala Val Ala Pro Val Ala Gly Ala Ile Glu Glu Ala Leu Ala Pro
   405 410 415
   Phe Gly Ala Arg Pro His Trp Gly Lys Val Phe Ser Thr Ala Pro Glu
   420 425 430
   Val Leu Arg Thr Leu Tyr Pro Arg Tyr Ala Asp Phe Glu Glu Leu Val
   435 440 445
   Gly Arg His Asp Pro Glu Gly Thr Phe Arg Asn Ala Phe Leu Asp Arg
   450 455 460
   Tyr Phe Arg Arg
   465
<210> 5
   <211> 1415
   <212> DNA
   <213> Artificial
<220>
   <223> Nucleotide seqeunce of fusion between signal seqeunce and SEQ ID No 1
<400> 5
   ccatgggcac cgaggtctcc cgccgcaagc tgatgaaggg cgcggcggtg tcgggcggcg 60
   cgctggcgct gccggccctc ggcgccccgc ccgccaccgc ggcgccggcc gccggccccg 120
   aggacctccc gggccccgcc gccgccatga ccccggccga gaagaactgg gccggcaaca 180
   tcaccttcgg cgccaagcgc ctgtgcgtcc cgcgctccgt ccgcgagctg cgcgagaccg 240
   tggccgcctc cggcgccgtg cgcccgctgg gcacccgcca ctcgttcaac accgtcgccg 300
   acacctccgg cgaccacgtg tcgctggccg gcctgccgcg cgtcgtcgac atcgacgtcc 360
   cgggccgggc cgtgtccctg tccgccggcc tgcgcttcgg cgagttcgcc gccgagctgc 420
   acgcccgcgg cctggccctg gccaacctgg gctccctgcc gcacatctcc gtggcgggcg    480
   cggtcgccac cggcacccac ggctccggcg tcggcaaccg ctccctggcg ggcgccgtcc    540
   gcgccctgtc cctggtcacc gccgacggcg agacccgcac cctgcgccgc accgacgagg    600
   acttcgccgg cgccgtcgtg tccctgggcg ccctgggcgt cgtcacctcc ctggagctgg    660
   acctggtccc ggccttcgag gtccgccagt gggtctacga ggacctgccc gaggccaccc    720
   tggccgcccg cttcgacgag gtcatgtccg ccgcctactc cgtgtccgtg ttcaccgact    780
   ggcgcccggg cccggtcggc caggtctggc tgaagcagcg cgtcggcgac gagggcgccc    840
   gctccgtcat gccggccgag tggctgggcg cccgcctggc cgacggcccg cgccacccgg    900
   tccccggcat gcccgccggc aactgcaccg cccagcaggg cgtcccgggc ccgtggcacg    960
   agcgcctgcc gcacttccgc atggagttca ccccgtccaa cggcgacgag ctgcagtccg   1020
   agtacttcgt cgcccgcgcg gacgccgtcg cggcctacga ggcgctggcc cgcctgcgcg   1080
   accgcatcgc cccggtcctg caggtctccg agctgcgcac cgtcgccgcc gacgacctgt   1140
   ggctgtcccc ggcccacggc cgcgactccg tcgccttcca cttcacctgg gtcccggacg   1200
   ccgccgccgt cgccccggtc gccggcgcca tcgaggaggc cctggccccg ttcggcgccc   1260
   gcccgcactg gggcaaggtg ttctccaccg cccccgaggt cctgcgcacc ctgtacccgc   1320
   gctacgccga cttcgaggag ctggtcggcc gccacgaccc cgagggcacc ttccgcaacg   1380
   ccttcctcga ccgctacttc cgccgctgag gatcc                              1415
<210> 6
   <211> 52
   <212> DNA
   <213> artificial
<220>
   <223> PCR primer
<400> 6
   gcgctagccg gccccccggc acaggccatg accccggccg agaagaactg gg 52
<210> 7
   <211> 17
   <212> DNA
   <213> artificial
<220>
   <223> PCR primer
<400> 7
   caggaaacag ctatgac 17
<210> 8
   <211> 1370
   <212> DNA
   <213> Nucleotide sequence encoding fusion between signal seqeunce and SEQ ID No 1 with introduced RE sites
<400> 8
   gcgctagccg gccccccggc acaggccatg accccggccg agaagaactg ggccggcaac      60
   atcaccttcg gcgccaagcg cctgtgcgtc ccgcgctccg tccgcgagct gcgcgagacc     120
   gtggccgcct ccggcgccgt gcgcccgctg ggcacccgcc actcgttcaa caccgtcgcc     180
   gacacctccg gcgaccacgt gtcgctggcc ggcctgccgc gcgtcgtcga catcgacgtc     240
   ccgggccggg ccgtgtccct gtccgccggc ctgcgcttcg gcgagttcgc cgccgagctg     300
   cacgcccgcg gcctggccct ggccaacctg ggctccctgc cgcacatctc cgtggcgggc     360
   gcggtcgcca ccggcaccca cggctccggc gtcggcaacc gctccctggc gggcgccgtc     420
   cgcgccctgt ccctggtcac cgccgacggc gagacccgca ccctgcgccg caccgacgag     480
   gacttcgccg gcgccgtcgt gtccctgggc gccctgggcg tcgtcacctc cctggagctg     540
   gacctggtcc cggccttcga ggtccgccag tgggtctacg aggacctgcc cgaggccacc     600
   ctggccgccc gcttcgacga ggtcatgtcc gccgcctact ccgtgtccgt gttcaccgac     660
   tggcgcccgg gcccggtcgg ccaggtctgg ctgaagcagc gcgtcggcga cgagggcgcc     720
   cgctccgtca tgccggccga gtggctgggc gcccgcctgg ccgacggccc gcgccacccg     780
   gtccccggca tgcccgccgg caactgcacc gcccagcagg gcgtcccggg cccgtggcac     840
   gagcgcctgc cgcacttccg catggagttc accccgtcca acggcgacga gctgcagtcc     900
   gagtacttcg tcgcccgcgc ggacgccgtc gcggcctacg aggcgctggc ccgcctgcgc     960
   gaccgcatcg ccccggtcct gcaggtctcc gagctgcgca ccgtcgccgc cgacgacctg    1020
   tggctgtccc cggcccacgg ccgcgactcc gtcgccttcc acttcacctg ggtcccggac    1080
   gccgccgccg tcgccccggt cgccggcgcc atcgaggagg ccctggcccc gttcggcgcc    1140
   cgcccgcact ggggcaaggt gttctccacc gcccccgagg tcctgcgcac cctgtacccg    1200
   cgctacgccg acttcgagga gctggtcggc cgccacgacc ccgagggcac cttccgcaac    1260
   gccttcctcg accgctactt ccgccgctga ggatccgagc tccagctttt gttcccttta    1320
   gtgagggtta attgcgcgct tggcgtaatc atggtcatag ctgtttcctg               1370
<210> 9
   <211> 27
   <212> DNA
   <213> artificial
<220>
   <223> PCR primer
<400> 9
   gcccatatga gcgacatcac ggtcacc 27
<210> 10
   <211> 24
   <212> DNA
   <213> artificial
<220>
   <223> PCR primer
<400> 10
   ggatcctcag cccgcgagca cccc 24
<210> 11
   <211> 27
   <212> DNA
   <213> artificial
<220>
   <223> PCR primer
<400> 11
   gccatgggcg acatcacggt caccaac 27
<210> 12
   <211> 26
   <212> DNA
   <213> artificial
<220>
   <223> PCR primer
<400> 12
   atggatcctc agcccgcgag cacccc 26
<210> 13
   <211> 1268
   <212> DNA
   <213> artificial
<220>
   <223> Streptomyces lividans and Streptomyces coelicolor SOX with the addition of the NCO1 cloning site at the start methionine
<400> 13
   gccatgggcg acatcacggt caccaactgg gccggcaaca tcacgtacac ggcgaaggaa 60
   ctgctgcggc cgcactccct ggacgcgctg cgggccctgg tggcggacag cgccagggtg 120
   cgggtgctgg gcagcgggca ctccttcaac gagatcgccg agccgggcga cgggggtgtc 180
   ctgctgtcgc tggcgggcct gccgtccgtg gtggacgtgg acacggcggc ccgtacggtg    240
   cgggtcggcg gcggtgtgcg gtacgcggag ctggcccggg tggtgcacgc gcggggcctg    300
   gcgctgccga acatggcctc gctgccgcac atctcggtcg ccgggtcggt ggccaccggc    360
   acccacggtt cgggggtggg caacggttcg ctggcctcgg tggtgcgcga ggtggagctg    420
   gtcaccgcgg acggttcgac cgtggtgatc gcgcggggcg acgagcggtt cggcggggcg    480
   gtgacctcgc tcggcgcgct gggcgtggtg acgtcgctca cactcgacct ggagccggcg    540
   tacgagatgg aacagcacgt cttcaccgag ctgccgctgg ccgggttgga cccggcgacg    600
   ttcgagacgg tgatggcggc ggcgtacagc gtgagtctgt tcaccgactg gcgggcgccc    660
   ggtttccggc aggtgtggct gaagcggcgc accgaccggc cgctggacgg tttcccgtac    720
   gcggccccgg ccgccgagaa gatgcatccg gtgccgggca tgcccgcggt gaactgcacg    780
   gagcagttcg ggtgccgggg gccctggcac gagcggctgc cgcacttccg cgcggagttc    840
   acgcccagca gcggtgccga gttgcagtcg gagtacctga tgccccggga gcacgccctg    900
   gccgccctgc acgcgatgga cgcgatacgg gagacgctcg cgccggtgct ccagacctgc    960
   gagatccgca cggtcgccgc cgacgcgcag tggctgagcc cggcgtacgg gcgggacacc   1020
   gtggccgcgc acttcacctg ggtcgaggac acggcggcgg tgctgccggt ggtgcggcgg   1080
   ctggaggagg cgctcgtccc cttcgcggcc cgtccgcact gggggaaggt gttcaccgtc   1140
   ccggcgggcg agctgcgtgc gctgtacccg cggctggccg acttcggggc gctggccggg   1200
   gcgctggacc cggcggggaa gttcaccaac gcgttcgtgc gcggggtgct cgcgggctga   1260
   ggatccat                                                             1268